# EUROPEAN PATENT APPLICATION

(11) **EP 3 782 982 A1**
(43) Date of publication of application: **24.02.2021**
(21) Application number: 20165313.6
(22) Date of filing: 24.03.2020
(51) Int. Cl.: C07C 303/36, C07C 311/48, C07C 303/40

(54) **PROCESS FOR THE SYNTHESIS OF FLUORINATED CONDUCTIVE SALTS FOR LITHIUM ION BATTERIES**

(30) Priority: 22.08.2019 DE 102019122656
(71) Applicant: Fujian Yongjing Technology Co., Ltd., Nanping, Fujian 354003 (CN)
(72) Inventor: CUI, Weilong, NANPING City, Fujian 354003 (CN); ZHOU, Changyue, NANPING City, Fujian 354003 (CN); DU, Hongjun, NANPING City, Fujian 354003 (CN); WU, Wenting, NANPING City, Fujian 354003 (CN)
(74) Representative: Gong, Jinping

(57) **Abstract**

The invention relates to a new process for the synthesis of fluorinated conductive salts for lithium ion batteries (Li-ion batteries). The said fluorinated conductive lithium ion (Li-ion) battery salts of interest in the framework of the present inventions synthesis process, for example, are Li-ion salts such as LiFSI (lithium bis-(fluoromethanesulfonlyl) imide), LiTFSI (lithium bis-(trifluormethanesulfonlyl) imide), and LiTFSFI (lithium trifluoromethanesulfonyl fluorosulfonyl imide), with the formulas as displayed in the Table I herein below. LiFSI, LiTFSI and LiFSTFSI are the most promising conducting salts for Lithium ion batteries and essential for future electromobility.

## Description

The invention relates to a new process for the synthesis of fluorinated conductive salts for lithium ion batteries (Li-ion batteries). The said fluorinated conductive lithium ion (Li-ion) battery salts of interest in the framework of the present inventions synthesis process, for example, are Li-ion salts such as LiFSI (lithium bis-(fluoromethanesulfonlyl) imide), LiTFSI (lithium bis-(trifluormethanesulfonlyl)imide), and LiTFSFI (lithium trifluoromethanesulfonylfluorosulfonylimide), with the formulas as displayed in the Table I herein below.

LiFSI, LiTFSI and LiFSTFSI are the most promising conducting salts for Lithium ion batteries and essential for future electromobility as commonly used conducting salts like LiPF₆ have drawbacks regarding stability and as consequence regarding safety of a battery. High purities of 99.9 % or even higher are necessary to achieve high live time and many re-charging cycles for securing an economic lifetime of the battery. As of today, there are several published synthesis methods but none of them allow economic synthesis which would allow large industrial scale production. The needed criteria for industrial scale are:
- either suitable fluorinated precursor or
- challenging fluorination step in late stage
- handling (or if possible better avoiding) of hazardous chemicals like HF, F₂, SO₃, COCl₂
- sophisticated purification step especially if fluorinating agents (especially with amine/amine/N-atom like structures) are used
- metal organic chemistry (high pure chemicals purification)

**Table I:**

| Fluorinated conductive salts for lithium ion batteries (Li-ion batteries) | | |
|---|---|---|
| LiFSI | lithium bis-(fluormethanesulfonlyl) imide | 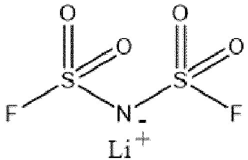 |
| | (Li bis(fluormethanesulfonlylimide) salt) | |
| LiTFSI | lithium bis-(trifluormethanesulfonlyl) imide | 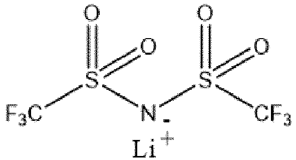 |
| | (Li bis(trifluormethanesulfonlyl) imide) salt) | |
| LiTFSFI | lithium trifluoromethane-sulfonylfluorosulfonylimide | 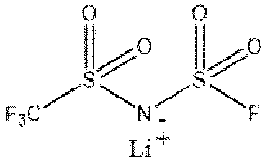 |
| | (Li trifluoromethanesulfonylfluorosulfonylimide salt) | |

Sate of the art synthesis routes are e.g. described by Central Glass (WO2016/080384, DE19533711), Lonza (WO2017/080831), Nippon Shokubai (WO2009/123328) and Phostech Lithium in US 7919629 .

In Lonza's and Nippon Shokubai's application, challenging fluorination has to happen in late stage. If this late stage fluorination step is done with a fluorinating agent and NOT with HF or F₂ etc. which need NO carrier of the "F"-atom (fluorinating agents always have a part of the molecule which need to be incinerated or at least which is very difficult to recyle), besides selectivity, purification is difficult, challenging or even impossible in view to economics. Using KF (or other metal fluorides "look" simple on first view, but as reactivity of such metal fluorides strongly depends on necessary dipolar aprotic solvents often in presence of crown ethers, ionic liquids or phase transfer catalysts etc. which cannot or only very difficult be separated from desired products. Other for standard organic chemists (but with no deep knowledge in fluorination chemistry) like pyridine/HF compounds (Olah's reagent) or simple amine x nHF agents has the advantage of easier handling but as the desired sulfonyl iminium salts are of similar structure, separation is impossible or again very challenging.

Regarding LiFSI-synthesis it has to be mentioned that due to reactivity, CISO₂NH₂ does NOT react with a 2nd SO₂Cl₂ to the Bis-Imide, so the much more reactive SO₂ClF described hereunder is a very suitable starting material with sufficient chemical reactivity. It was found that SO₂ClF can be made selectively without any catalyst by just starting with SO₂Cl₂ and a feed of anhydrous HF to generate "just" HCl which is a sales product. Up to now, as known, the synthesis of SO₂FCl is only described in presence of a fluorination catalyst/agent like DBN x HF like in WO 02/06083 but never without any catalyst. This reaction procedure also has the advantage that NO fluorination step is needed to be applied in the Bis-Imide form where needed purification to the required quality for battery applications is almost impossible.

The object of the present invention is to overcome or to essentially avoid the disadvantages of the prior art, and to provide an advantageous process for the manufacture of fluorinated conductive salts for lithium ion batteries (Li-ion batteries) as shown in Table I above, preferably to provide an advantageous process for the manufacture of LiFSI (lithium bis-(fluormethanesulfonlyl) imide; Li bis(fluormethanesulfonlyl imide) salt), as the fluorinated conductive salts for lithium ion batteries (Li-ion batteries). Especially, the said fluorinated conductive salts for lithium ion batteries (Li-ion batteries) shall be provided with high purity. Hence, it is also an object of the invention to give very good yields, and to allow for large scale and/or industrial production and to avoid challenging purification problems of the prior art.

The objects of the invention are solved as defined in the claims, and described herein after in detail. The invention disclosed hereunder gives very good yields, and allows for large scale and/or industrial production and to avoid challenging purification problems observed in the prior art.

Regarding the scope of the present invention it is to be noted that, that for legal reason only but not for technical reason, there is a proviso that any procedure or manufacture of a fluorinated, direct or indirect, precursor compound involving a direct fluorination of compounds with highly concentrated fluorine gas, unless expressively otherwise stated herein, and fluorinated compounds as such being directly obtained from such direct fluorination of compounds with highly concentrated fluorine gas are exempted from the scope of the present invention.

However, to be clear, in contrast to the above, the use a fluorinated, direct or indirect, precursor compound being directly or indirectly obtained by a direct fluorination of compounds with highly concentrated fluorine gas, i.e. the use in a process for the manufacture of fluorinated conductive salts for lithium ion batteries (Li-ion batteries) is expressively encompassed by the present invention.

A highly concentrated fluorine gas is to be understood for the purpose of this invention to be a fluorine gas (fluorination gas) with concentrations of substantially more than, in particular very much higher than 15 % by volume or in particular than 20 % by volume of elemental fluorine (F₂), especially of equal to much higher than 25 % by volume (i.e., at least 25 % by volume) of elemental fluorine (F₂), preferably of equal to much higher than 35 % by volume or in particular than 45 % by volume of elemental fluorine (F₂), that is used for chemical synthesis, especially for the manufacture or for preparation of a fluorinated inorganic compound or fluorinated organic compound, respectively, as final products and/or intermediates.

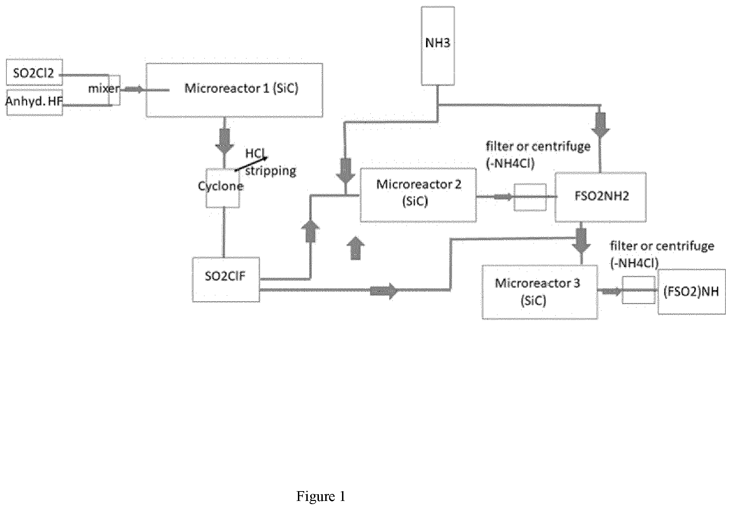

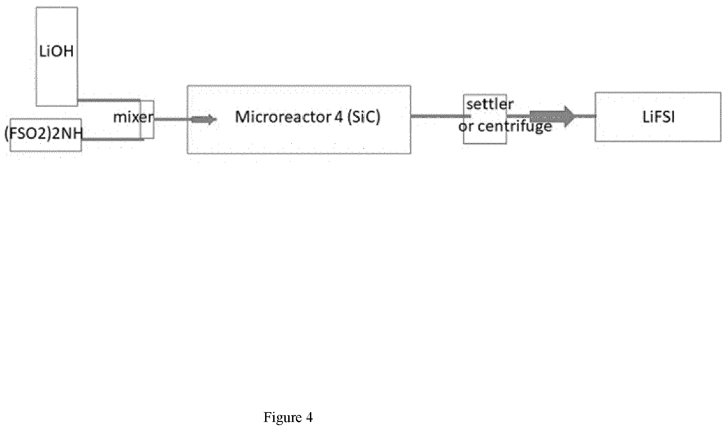

The present invention shall be described in detail in view of embodiments, and in this context particular reference is also made to the Examples and Reaction Schemes shown therein, as well as to the Figures 1 to 4.

In one aspect the invention preferably relates to the most important conductive salt "LiFSI" (lithium bis-(fluormethanesulfonlyl) imide; Li bis(fluormethanesulfonlyl imide) salt).

A further aspect of the invention preferably relates to a process for the manufacture of fluorinated conductive salts for lithium ion batteries (Li-ion batteries) as shown in Table I above, preferably to provide an advantageous process for the manufacture of LiFSI (lithium bis-(fluormethanesulfonlyl) imide; Li bis(fluormethanesulfonlyl imide) salt), as the fluorinated conductive salts for lithium ion batteries (Li-ion batteries), wherein the production of conductive salts for lithium ion batteries (Li-ion batteries) takes place, directly or indirectly, via a fluorinated sulfonyl compound having the formula (III),F-SO₂₋Cl (III) (fluorosulfuric acid chloride); herein, the group -SO₂- is named "sulfonyl group".

It was observed by the present invention that the fluorinated sulfonyl compound having the formula (III),F-SO₂₋Cl (III) (fluorosulfuric acid chloride), is much more reactive with amides than the corresponding difluoride SO₂F₂.

In still a further aspect the invention preferably relates to a process for the manufacture of fluorinated conductive salts for lithium ion batteries (Li-ion batteries) as shown in Table I above, preferably to provide an advantageous process for the manufacture of LiFSI (lithium bis-(fluormethanesulfonlyl) imide; Li bis(fluormethanesulfonlyl imide) salt), as the fluorinated conductive salts for lithium ion batteries (Li-ion batteries), wherein the production of conductive salts for lithium ion batteries (Li-ion batteries) takes place, at least in one reaction step a continuous flow reactor, and more preferably in a microreactor.

Also, the reactions, in particular in continuous flow reactor or more preferably in a microreactor, is performed without requiring a catalyst.

In another aspect the invention preferably relates to a process for the manufacture of fluorinated conductive salts for lithium ion batteries (Li-ion batteries) as shown in Table I above, preferably to provide an advantageous process for the manufacture of LiFSI (lithium bis-(fluormethanesulfonlyl) imide; Li bis(fluormethanesulfonlyl imide) salt), as the fluorinated conductive salts for lithium ion batteries (Li-ion batteries), wherein the production of a bis-imide, as defined herein, is performed directly from the corresponding fluorosulfonic acid amide, and, for example, not via an isocyanate as an additional stage as in Lonza's process. However, if occasionally the reaction rate in an industrial plant should be too low, the isocyanate intermediate could be envisaged as a fallback procedure.

The following embodiments are intended to further describe the aspects, i.e., the processes and the uses, of the invention.

In embodiment 1, the invention relates to a process for the manufacture of a fluorinated bis-sulfonyl imide of formula (I), preferably as precursor for a conductive lithium salt of the corresponding fluorinated sulfonyl imide of formula (II) for use in a lithium battery application, 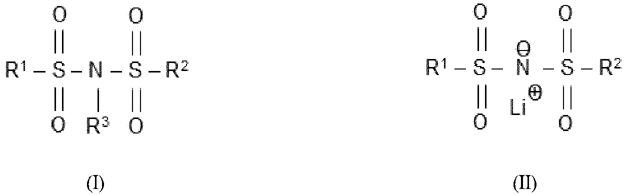 wherein in formula (I) and in formula (II),
R1 and R2 independently denote fluorine (F) or a trifluormethyl group, and
R3 denotes hydrogen (H), fluorine (F), or R3 denotes an electron pair forming together with the nitrogen (N) negatively charged nitrogen (N⁻) an to which in turn a positively charged tri-alkyl ammonium group is ionically bound, preferably a tri-ethyl ammonium group;
wherein the process comprises, at least one or a combination of the following:
(i) providing as a first direct or indirect precursor compound of a fluorinated bis-sulfonyl imide of formula (I), a fluorinated sulfonyl compound having the formula (III),

   F-SO₂-Cl (III)

   , (fluorosulfuric acid chloride), or a fluorinated sulfonyl compound selected from the group consisting of a compound having one of formulae (IV), (V) or (VI), with the proviso that the said one compound of formulae (IV), (V) or (VI) is directly obtained from the fluorinated sulfonyl compound having the formula (III),

   F-SO₂-OH (IV),

   CF₃-SO₂-OH (V),

   CF₃-SO₂-Cl (VI);

   and
(ii) providing as a second precursor compound of a fluorinated bis-sulfonyl imide of formula (I), a compound selected from the group consisting of a sulfonyl amino compound (sulfuric acid amide compound) a) to c), or a sulfonyl isocyanate compound d) to e):
   a) F-SO₂-NH₂ (VII) derived from F-SO₂-NH₂ x NH4F, obtained by reacting F-SO₂-Cl with ammonia (NH₃), or
   b) F₃C-SO₂-NH₂ (VIII) derived from F₃C-SO₂-NH₂ x NH₄F, obtained by reacting F₃C-SO₂-Cl with ammonia (NH₃), or
   c) F₃C-SO₂-NH₂ (VIII) derived from F₃C-SO₂-NH₂ x NH₄F, obtained by reacting F₃C-SO₂-F (TFMSAF, trifluoromethylsulfuric acid fluoride) with ammonia (NH₃),
   d) F-SO₂-N=C=O (IX), obtained by reacting F-CN with SO₃, or
   e) F₃C-SO₂-N=C=O (X), obtained by reacting CF₃-CN with SO₃;
(iii) reacting a first precursor compound of (i) with a second precursor compound of (ii), each of the a fluorinated bis-sulfonyl imide of formula (I), with the proviso that the second precursor compound of (ii),
   (1) in case of a), b) and c) the compound (VII) of a), the compound (VII) of b), or the compound (VII) of c) is reacted either with the fluorinated sulfonyl compound having the formula (III), F-SO₂₋Cl, or with the fluorinated sulfonyl compound having the formula (the fluorinated sulfonyl compound having the formula (VI), CF₃-SO₂₋Cl,
      to obtain a fluorinated bis-sulfonyl imide of formula (I), wherein R3 denotes hydrogen (H), and R1 and R2 independently denote fluorine (F) or a trifluormethyl group;
      or
   (2) in case of d) and e), the compound (IX) of d) or the compound (X) of e) is reacted either with the fluorinated sulfonyl compound having the formula (IV), F-SO₂-OH, or with the fluorinated sulfonyl compound having the formula (the fluorinated sulfonyl compound having the formula (V), CF₃-SO₂-OH,
      to obtain a fluorinated bis-sulfonyl imide of formula (I), wherein R3 denotes hydrogen (H), and R1 and R2 independently denote fluorine (F) or a trifluormethyl group;
      or
   (3) in case of a), b) and c) the compound (VII) of a), the compound (VII) of b), or the compound (VII) of c) is
      reacted with NEt₃ and TFMSAF (trifluoromethylsulfuric acid fluoride)
      to obtain a fluorinated bis-sulfonyl imide of formula (I), wherein R3 denotes an electron pair forming together with the nitrogen (N) negatively charged nitrogen (N⁻) an to which in turn a positively charged tri-alkyl ammonium group is ionically bound, preferably a tri-ethyl ammonium group, preferably wherein R3 is NEt₃ and the fluorinated bis-sulfonyl imide of formula (I) is bis-TFMSAA x NEt₃;
      and optionally
   (4) further reacting the fluorinated bis-sulfonyl imide of formula (I) obtained under (1) or (2), wherein R3 is hydrogen (H), with concentrated elemental fluorine (F₂) in hydrogen fluoride (HF) or concentrated elemental fluorine (F₂) in an inert solvent, for exampleacetonitrile,
      to obtain a fluorinated bis-sulfonyl imide of formula (I), wherein R3 denotes fluorine (F);
      and optionally
   (5) converting the fluorinated bis-sulfonyl imide of formula (I), obtained under any of (1) to (4) into a conductive lithium salt of the corresponding fluorinated sulfonyl imide of formula (II), preferably the sulfonyl imide of formula (II) for use in a lithium battery application, wherein R1 and R2 independently denote fluorine (F) or a trifluormethyl group.

In embodiment 2, the invention relates to a process according to embodiment 1, for the manufacture of a fluorinated bis-sulfonyl imide of formula (I), wherein the process is carried out without a catalyst.

In embodiment 3, the invention relates to a process according to embodiment1 or embodiment2, for the manufacture of a fluorinated bis-sulfonyl imide of formula (I), wherein the process is carried out at least in one step in a microreactor.

In embodiment 4, the invention relates to a process according to any of the preceding embodiments, for the manufacture of a fluorinated bis-sulfonyl imide of formula (I), wherein the process is carried out with a first direct or indirect precursor compound of the a fluorinated bis-sulfonyl imide of formula (I), which is a fluorinated sulfonyl compound having the formula (III),

F-SO₂-Cl (III),

(fluorosulfuric acid chloride).

In embodiment 3, the invention relates to a process according to any of the preceding embodiments, for the manufacture of a fluorinated bis-sulfonyl imide of formula (I), wherein the process is carried out in step (ii) of claim 1 by providing as a second precursor compound of the a fluorinated bis-sulfonyl imide of formula (I), a compound selected from the group consisting of a sulfonyl amino compound (sulfuric acid amide compound) a) to c).

In embodiment 6, the invention relates to a process according to any of embodiment1 to embodiment4, for the manufacture of a fluorinated bis-sulfonyl imide of formula (I), wherein the process is carried out in step (ii) of embodiment1 by providing as a second precursor compound of the a fluorinated bis-sulfonyl imide of formula (I), a compound selected from the group consisting of a sulfonyl isocyanate compound d) to e).

In embodiment7, the invention relates to a process according to any of the preceding embodiments, for the manufacture of a fluorinated bis-sulfonyl imide of formula (I), wherein the fluorinated bis-sulfonyl imide of formula (I), is selected from the group consisting of:

| | | |
|---|---|---|
| LiFSI | lithium bis-(fluoromethanesulfonlyl) imide | 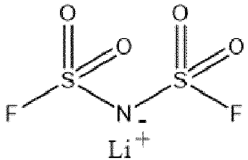 |
| | (Li bis(fluoromethanesulfonlylimide) salt) | |
| LiTFSI | lithium bis-(trifluoromethanesulfonlyl) imide | 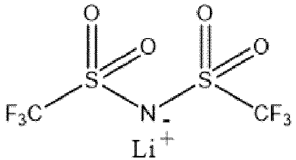 |
| | (Li bis-(trifluoromethanesulfonlyl) imide) salt) | |
| LiTFSFI | lithium trifluoromethanesulfonylfluorosulfonyl imide | 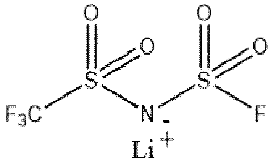 |
| | (Li trifluoromethanesulfonylfluorosulfonyl imide salt) | |

In embodiment 8, the invention relates to a process according to any of embodiment1 to embodiment7, for the manufacture of a fluorinated bis-sulfonyl imide of formula (I), wherein the fluorinated bis-sulfonyl imide of formula (I), is lithium bis-(fluoromethanesulfonlyl) imide (LiFSI, Li bis-(fluoromethanesulfonlyl imide) salt).

In embodiment9, the invention relates to aprocess for the manufacture of a fluorinated compound according to any of embodiments 1 to 8, wherein the reaction is carried out in at least one step as acontinuous processes, wherein the continuous process is performed in at least one continuous flow reactor with upper lateral dimensions of about ≤ 5 mm, or of about ≤ 4 mm,
preferably in at least one microreactor;
more preferably wherein of the said steps at least (b2) the step of a fluorination reaction is a continuous process in at least one microreactor under one or more of the following conditions:
- flow rate: of from about 10 ml/h up to about 400 1/h;
- temperature: of from about 30 °C up to about 150 °C;
- pressure: of from about 4 bar up to about 50 bar;
- residence time: of from about 1 second, preferably from about 1 minute, up to about 60 minutes.

In embodiment 10, the invention relates to aprocess of preparing a compound according to embodiment9, wherein at least one of the said continuous flow reactors, preferably at least one of the microreactors, independently is a SiC-continuous flow reactor, preferably independently is an SiC-microreactor.

In embodiment 11, the invention relates to a use of a precursor compound selected from the group consisting of:
(i) as a first direct or indirect precursor compound (1-i) or (1-ii) of a fluorinated bis-sulfonyl imide of formula (I);
   (i-1) a fluorinated sulfonyl compound having the formula (III),

      F-SO₂-Cl (III),

      (fluorosulfuric acid chloride); or
   (i-2) a fluorinated sulfonyl compound selected from the group consisting of a compound having one of formulae (IV), (V) or (VI), with the proviso that the said one compound of formulae (IV), (V) or (VI) is directly obtained from the fluorinated sulfonyl compound having the formula (III),

      F-SO₂-OH (IV),

      CF₃-SO₂-OH (V),

      CF₃-SO₂-Cl (VI);
   or
(ii) as a second precursor compound of a fluorinated bis-sulfonyl imide of formula (I),a compound selected from the group consisting of a sulfonyl amino compound (sulfuric acid amide compound) a) to c), or a sulfonyl isocyanate compound d) to e):
   a) F-SO₂-NH₂ (VII) derived from F-SO₂-NH₂ x NH4F, obtained by reacting F-SO₂-Cl with ammonia (NH₃), or
   b) F₃C-SO₂-NH₂ (VIII) derived from F₃C-SO₂-NH₂ x NH₄F, obtained by reacting F₃C-SO₂-Cl with ammonia (NH₃), or
   c) F₃C-SO₂-NH₂ (VIII) derived from F₃C-SO₂-NH₂ x NH₄F, obtained by reacting F₃C-SO₂-F (TFMSAF, trifluoromethylsulfuric acid fluoride) with ammonia (NH₃),
   d) F-SO₂-N=C=O (IX), obtained by reacting F-CN with SO₃, or
   e) F₃C-SO₂-N=C=O (X), obtained by reacting CF₃-CN with SO₃;
   in a process for the manufacture of a fluorinated bis-sulfonyl imide of formula (I), preferably as precursor for a conductive
lithium salt of the corresponding fluorinated sulfonyl imide of formula (II) for use in a lithium battery application, 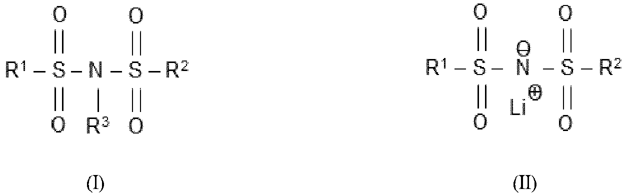 wherein in formula (I) and in formula (II),
R1 and R2 independently denote fluorine (F) or a trifluormethyl group, and
R3 denotes hydrogen (H), fluorine (F), or R3 denotes an electron pair forming together with the nitrogen (N) negatively charged nitrogen (N⁻) an to which in turn a positively charged tri-alkyl ammonium group is ionically bound, preferably a tri-ethyl ammonium group.

In embodiment 12, the invention relates to a use of a precursor compound according to embodiment 11, in a process for the manufacture of a fluorinated bis-sulfonyl imide of formula (I), preferably as precursor for a conductive lithium salt of the corresponding fluorinated sulfonyl imide of formula (II) for use in a lithium battery application,
wherein the use of at least one precursor compound in the process for the manufacture of a fluorinated compound of formula (I) or formula (II), as each defined in embodiment 11, takes place in a reaction which is carried out in at least one step as a continuous processes, wherein the continuous process is performed in at least one continuous flow reactor with upper lateral dimensions of about ≤ 5 mm, or of about ≤ 4 mm,
preferably in at least one microreactor;
more preferably wherein of the said steps at least (b2) the step of a fluorination reaction is a continuous process in at least one microreactor under one or more of the following conditions:
- flow rate: of from about 10 ml/h up to about 400 1/h;
- temperature: of from about 30 °C up to about 150 °C;
- pressure: of from about 4 bar up to about 50 bar;
- residence time: of from about 1 second, preferably from about 1 minute, up to about 60 minutes.

In embodiment 13, the invention relates to a use of a precursor compound according to embodiment 12, wherein the use of at least one precursor compound in the process for the manufacture of a fluorinated compound of formula (I) or formula (II), as each defined in embodiment 11, takes place in a continuous process, wherein at least one of the said continuous flow reactors, preferably at least one of the microreactors, independently is a SiC-continuous flow reactor, preferably independently is an SiC-microreactor.

### Microreactor Process in the Invention:

The invention also may pertain to a process for the manufacture according to the invention, wherein the process is a continuous process, preferably wherein the continuous process is carried out in a microreactor. See Figures .

The invention may employ more than a single microreactor, i.e., the invention may employ two, three, four, five or more microreactors, for either extending the capacity or residence time, for example, to up to ten microreactors in parallel or four microreactors in series. If more than a single microreactor is employed, then the plurality of microreactors can be arranged either sequentially or in parallel, and if three or more microreactors are employed, these may be arranged sequentially, in parallel or both.

The invention is also very advantageous, in one embodiment wherein the process of the invention optionally is performed in a continuous flow reactor system, or preferably in a microreactor system.

In an preferred embodiment the invention relates to a process according to the invention, wherein the reaction is carried out in at least one step as acontinuous processes, wherein the continuous process is performed in at least one continuous flow reactor with upper lateral dimensions of about ≤ 5 mm, or of about ≤ 4 mm,
preferably in at least one microreactor;
more preferably wherein of the said steps at least one step is a continuous process in at least one microreactor under one or more of the following conditions:
- flow rate: of from about 10 ml/h up to about 400 1/h;
- temperature: of from about 30 °C up to about 150 °C;
- pressure: of from about 4 bar up to about 50 bar;
- residence time: of from about 1 second, preferably from about 1 minute, up to about 60 minutes.

In another preferred embodiment the invention relates to such a process according to the invention, wherein at least one of the said continuous flow reactors, preferably at least one of the microreactors, independently is a SiC-continuous flow reactor, preferably independently is an SiC-microreactor.

### The Continuous Flow Reactors and Microreactors:

In addition to the above, according to one aspect of the invention, also a plant engineering invention is provided, as used in the process invention and described herein, pertaining to the optional, and in some embodiments of the process invention, the process even preferred implementation in microreactors.

As to the term "microreactor": A "microreactor" or "microstructured reactor" or "microchannel reactor", in one embodiment of the invention, is a device in which chemical reactions take place in a confinement with typical lateral dimensions of about ≤ 1 mm; an example of a typical form of such confinement are microchannels. Generally, in the context of the invention, the term "microreactor": A "microreactor" or "microstructured reactor" or "microchannel reactor", denotes a device in which chemical reactions take place in a confinement with typical lateral dimensions of about ≤ 5 mm

Microreactors are studied in the field of micro process engineering, together with other devices (such as micro heat exchangers) in which physical processes occur. The microreactor is usually a continuous flow reactor (contrast with/to a batch reactor). Microcreactors offer many advantages over conventional scale reactors, including vast improvements in energy efficiency, reaction speed and yield, safety, reliability, scalability, on-site/on-demand production, and a much finer degree of process control.

Microreactors are used in "flow chemistry" to perform chemical reactions.

In flow chemistry, wherein often microreactors are used, a chemical reaction is run in a continuously flowing stream rather than in batch production. Batch production is a technique used in manufacturing, in which the object in question is created stage by stage over a series of workstations, and different batches of products are made. Together with job production (one-off production) and mass production (flow production or continuous production) it is one of the three main production methods. In contrast, in flow chemistry the chemical reaction is run in a continuously flowing stream, wherein pumps move fluid into a tube, and where tubes join one another, the fluids contact one another. If these fluids are reactive, a reaction takes place. Flow chemistry is a well-established technique for use at a large scale when manufacturing large quantities of a given material. However, the term has only been coined recently for its application on a laboratory scale.

Continuous flow reactors, e.g. such as used as microreactor, are typically tube like and manufactured from non-reactive materials, such known in the prior art and depending on the specific purpose and nature of possibly aggressive agents and/or reactants. Mixing methods include diffusion alone, e.g. if the diameter of the reactor is narrow, e.g. < 1 mm, such as in microreactors, and static mixers. Continuous flow reactors allow good control over reaction conditions including heat transfer, time and mixing. The residence time of the reagents in the reactor, i.e. the amount of time that the reaction is heated or cooled, is calculated from the volume of the reactor and the flow rate through it: Residence time = Reactor Volume / Flow Rate. Therefore, to achieve a longer residence time, reagents can be pumped more slowly, just a larger volume reactor can be used and/or even several microreactors can be placed in series, optionally just having some cylinders in between for increasing residence time if necessary for completion of reaction steps. In this later case, cyclones after each microreactor help to let formed HCl to escape and to positively influence the reaction performance. Production rates can vary from milliliters per minute to liters per hour.

Some examples of flow reactors are spinning disk reactors (Colin Ramshaw); spinning tube reactors; multi-cell flow reactors; oscillatory flow reactors; microreactors; hex reactors; and aspirator reactors. In an aspirator reactor a pump propels one reagent, which causes a reactant to be sucked in. Also to be mentioned are plug flow reactors and tubular flow reactors.

In the present invention, in one embodiment it is particularly preferred to employ a microreactor.

In the use and processes according to the invention in a preferred embodiment the invention is using a microreactor. But it is to be noted in a more general embodiment of the invention, apart from the said preferred embodiment of the invention that is using a microreactor, any other, e.g. preferentially pipe-like, continuous flow reactor with upper lateral dimensions of up to about 1 cm, and as defined herein, can be employed. Thus, such a continuous flow reactor preferably with upper lateral dimensions of up to about ≤ 5 mm, or of about ≤ 4 mm, refers to a preferred embodiment of the invention, e.g. preferably to a microreactor. Continuously operated series of STRs is another option, but less preferred than using a microreactor.

In the before said embodiments of the invention, the minimal lateral dimensions of the, e.g. preferentially pipe-like, continuous flow reactor can be about > 5 mm; but is usually not exceeding about 1 cm. Thus, the lateral dimensions of the, e.g. preferentially pipe-like, continuous flow reactor can be in the range of from about > 5 mm up to about 1 cm, and can be of any value therein between. For example, the lateral dimensions of the, e.g. preferentially pipe-like, continuous flow reactor can be about 5.1 mm, about 5.5 mm, about 6 mm, about 6.5 mm, about 7 mm, about 7.5 mm, about 8 mm, about 8.5 mm, about 9 mm, about 9.5 mm, and about 10 mm, or can be can be of any value intermediate between the said values.

In the before said embodiments of the invention using a microreactor preferentially the minimal lateral dimensions of the microreactor can be at least about 0.25 mm, and preferably at least about 0.5 mm; but the maximum lateral dimensions of the microreactor does not exceed about ≤ 5 mm. Thus, the lateral dimensions of the, e.g. preferential microreactor can be in the range of from about 0.25 mm up to about ≤ 5 mm, and preferably from about 0.5 mm up to about ≤ 5 mm, and can be of any value therein between. For example, the lateral dimensions of the preferential microreactor can be about 0.25 mm, about 0.3 mm, about 0.35 mm, about 0.4 mm, about 0.45 mm, and about 5 mm, or can be can be of any value intermediate between the said values.

As stated here before in the embodiments of the invention in its broadest meaning is employing, preferentially pipe-like, continuous flow reactor with upper lateral dimensions of up to about 1 cm. Such continuous flow reactor, for example is a plug flow reactor (PFR).

The plug flow reactor (PFR), sometimes called continuous tubular reactor, CTR, or piston flow reactors, is a reactor used to perform and describe chemical reactions in continuous, flowing systems of cylindrical geometry. The PFR reactor model is used to predict the behavior of chemical reactors of such design, so that key reactor variables, such as the dimensions of the reactor, can be estimated.

Fluid going through a PFR may be modeled as flowing through the reactor as a series of infinitely thin coherent "plugs", each with a uniform composition, traveling in the axial direction of the reactor, with each plug having a different composition from the ones before and after it. The key assumption is that as a plug flows through a PFR, the fluid is perfectly mixed in the radial direction (i.e. in the lateral direction) but not in the axial direction (forwards or backwards).

Accordingly, the terms used herein to define the reactor type used in the context of the invention such like "continuous flow reactor", "plug flow reactor", "tubular reactor", "continuous flow reactor system", "plug flow reactor system", "tubular reactor system", "continuous flow system", "plug flow system", "tubular system" are synonymous to each other and interchangeably by each other.

The reactor or system may be arranged as a multitude of tubes, which may be, for example, linear, looped, meandering, circled, coiled, or combinations thereof. If coiled, for example, then the reactor or system is also called "coiled reactor" or "coiled system".

In the radial direction, i.e. in the lateral direction, such reactor or system may have an inner diameter or an inner cross-section dimension (i.e. radial dimension or lateral dimension, respectively) of up to about 1 cm. Thus, in an embodiment the lateral dimension of the reactor or system may be in the range of from about 0,25 mm up to about 1 cm, preferably of from about 0,5 mm up to about 1 cm, and more preferably of from about 1 mm up to about 1 cm.

In further embodiments the lateral dimension of the reactor or system may be in the range of from about > 5 mm to about 1 cm, or of from about 5.1 mm to about 1 cm.

If the lateral dimension at maximum of up to about ≤ 5 mm, or of up to about ≤ 4 mm, then the reactor is called "microreactor". Thus, in still further microreactor embodiments the lateral dimension of the reactor or system may be in the range of from about 0,25 mm up to about ≤ 5 mm, preferably of from about 0,5 mm up to about ≤ 5 mm, and more preferably of from about 1 mm up to about ≤ 5 mm; or the lateral dimension of the reactor or system may be in the range of from about 0,25 mm up to about ≤ 4 mm, preferably of from about 0,5 mm up to about ≤ 4 mm, and more preferably of from about 1 mm up to about ≤ 4 mm

In case reactants are solid inert solvents may be used. Thus, if raw materials shall be used, then the said solid raw materials are dissolved in an inert solvent. A suitable solvent is e.g. acetonitrile, or fully or partially fluorinated alkanes like Pentafluorobutane (365mfc), linear or cyclic partially or fully fluorinated ethers like CF₃-CH₂-OCHF₂ (E245) or Octafluorotetrahydrofuran. Often, if available or after a first synthesis, the product as such can also serve as inert solvent.

In an alternative embodiment of the invention, it is also optionally desired to employ another continuous flow reactor than a microreactor, preferably if, for example, the (halogenation promoting, e.g. the halogenation or preferably the halogenation) catalyst composition used in the halogenation or fluorination tends to get viscous during reaction or is viscous already as a said catalyst as such. In such case, a continuous flow reactor, i.e. a device in which chemical reactions take place in a confinement with lower lateral dimensions of greater than that indicated above for a microreactor, i.e. of greater than about 1 mm, but wherein the upper lateral dimensions are about ≤ 4 mm. Accordingly, in this alternative embodiment of the invention, employing a continuous flow reactor, the term "continuous flow reactor" preferably denotes a device in which chemical reactions take place in a confinement with typical lateral dimensions of from about ≥ 1 mm up to about ≤ 4 mm. In such an embodiment of the invention it is particularly preferred to employ as a continuous flow reactor a plug flow reactor and/or a tubular flow reactor, with the said lateral dimensions. Also, in such an embodiment of the invention, as compared to the embodiment employing a microreactor, it is particularly preferred to employ higher flow rates in the continuous flow reactor, preferably in the plug flow reactor and/or a tubular flow reactor, with the said lateral dimensions. For example, such higher flow rates, are up to about 2 times higher, up to about 3 times higher, up to about 4 times higher, up to about 5 times higher, up to about 6 times higher, up to about 7 times higher, or any intermediate flow rate of from about ≥ 1 up to about ≤ 7 times higher, of from about ≥ 1 up to about ≤ 6 times higher, of from about ≥ 1 up to about ≤ 5 times higher, of from about ≥ 1 up to about ≤ 4 times higher, of from about ≥ 1 up to about ≤ 3 times higher, or of from about ≥ 1 up to about ≤ 2 times higher, each as compared to the typical flow rates indicated herein for a microreactor. Preferably, the said continuous flow reactor, more preferably the plug flow reactor and/or a tubular flow reactor, employed in this embodiment of the invention is configured with the construction materials as defined herein for the microreactors. For example, such construction materials are silicon carbide (SiC) and/or are alloys such as a highly corrosion resistant nickel-chromium-molybdenum-tungsten alloy, e.g. Hastelloy®, as described herein for the microreactors.

A very particular advantage of the present invention employing a microreactor, or a continuous flow reactor with the before said lateral dimensions, the number of separating steps can be reduced and simplified, and may be devoid of time and energy consuming, e.g. intermediate, distillation steps. Especially, it is a particular advantage of the present invention employing a microreactor, or a continuous flow reactor with the before said lateral dimensions, that for separating simply phase separation methods can be employed, and the non-consumed reaction components may be recycled into the process, or otherwise be used as a product itself, as applicable or desired.

In addition to the preferred embodiments of the present invention using a microreactor according to the invention, in addition or alternatively to using a microreactor, it is also possible to employ a plug flow reactor or a tubular flow reactor, respectively.

Plug flow reactor or tubular flow reactor, respectively, and their operation conditions, are well known to those skilled in the field.

Although the use of a continuous flow reactor with upper lateral dimensions of about ≤ 5 mm, or of about ≤ 4 mm, respectively, and in particular of a microreactor, is particularly preferred in the present invention, depending on the circumstances, it could be imagined that somebody dispenses with an microreactor, then of course with yield losses and higher residence time, higher temperature, and instead takes a plug flow reactor or turbulent flow reactor, respectively. However, this could have a potential advantage, taking note of the mentioned possibly disadvantageous yield losses, namely the advantage that the probability of possible blockages (tar particle formation by non-ideal driving style) could be reduced because the diameters of the tubes or channels of a plug flow reactor are greater than those of a microreactor.

The possibly allegeable disadvantage of this variant using a plug flow reactor or a tubular flow reactor, however, may also be seen only as subjective point of view, but on the other hand under certain process constraints in a region or at a production facility may still be appropriate, and loss of yields be considered of less importance or even being acceptable in view of other advantages or avoidance of constraints.

In the following, the invention is more particularly described in the context of using a microreactor. Preferentially, a microreactor used according to the invention is a ceramic continuous flow reactor, more preferably an SiC (silicon carbide) continuous flow reactor, and can be used for material production at a multi-to scale. Within integrated heat exchangers and SiC materials of construction, it gives optimal control of challenging flow chemistry application. The compact, modular construction of the flow production reactor enables, advantageously for: long term flexibility towards different process types; access to a range of production volumes (5 to 400 1/h); intensified chemical production where space is limited; unrivalled chemical compatibility and thermal control.

Ceramic (SiC) microreactors, are e.g. advantageously diffusion bonded 3M SiC reactors, especially braze and metal free, provide for excellent heat and mass transfer, superior chemical compatibility, of FDA certified materials of construction, or of other drug regulatory authority (e.g. EMA) certified materials of construction. Silicon carbide (SiC), also known as carborundum, is a containing silicon and carbon, and is well known to those skilled in the art. For example, synthetic SiC powder is been mass-produced and processed for many technical applications.

For example, in the embodiments of the invention the objects are achieved by a method in which at least one reaction step takes place in a microreactor. Particularly, in preferred embodiments of the invention the objects are achieved by a method in which at least one reaction step takes place in a microreactor that is comprising or is made of SiC ("SiC-microreactor"), or in a microreactor that is comprising or is made of an alloy, e.g. such as Hastelloy C, as it is each defined herein after in more detail.

Thus, without being limited to, for example, in an embodiment of the invention the microreactor suitable for, preferably for industrial, production an "SiC-microreactor" that is comprising or is made of SiC (silicon carbide; e.g. SiC as offered by Dow Corning as Type G1SiC or by Chemtrix MR555 Plantrix), e.g. providing a production capacity of from about 5 up to about 400 kg per hour; or without being limited to, for example, in another embodiment of the invention the microreactor suitable for industrial production is comprising or is made of Hastelloy C, as offered by Ehrfeld. Such microreactors are particularly suitable for the, preferably industrial, production of fluorinated products according to the invention.

In order to meet both the mechanical and chemical demands placed on production scale flow reactors, Plantrixmodules are fabricated from 3M™SiC (Grade C). Produced using the patented 3M (EP 1 637 271 B1 and foreign patents) diffusion bonding technology, the resulting monolithic reactors are hermetically sealed and are free from welding lines/joints and brazing agents. More technical information on the Chemtrix MR555 Plantrix can be found in the brochure "CHEMTRIX - Scalable Flow Chemistry - Technical Information Plantrix® MR555 Series, published by Chemtrix BV in 2017, which technical information is incorporated herein by reference in its entirety.

Apart from the before said example, in other embodiments of the invention, in general SiC from other manufactures, and as known to the skilled person, of course can be employed in the present invention.

Accordingly, in the present invention as microreactor also the Protrix® of by Chemtrix can be used. Protrix® is a modular, continuous flow reactor fabricated from 3M® silicon carbide, offering superior chemical resistance and heat transfer. In order to meet both the mechanical and chemical demands placed on flow reactors, Protrix® modules are fabricated from 3M® SiC (Grade C). Produced using the patented 3M (EP 1 637 271 B1 and foreign patents) diffusion bonding technology, the resulting monolithic reactors are hermetically sealed and are free from welding lines/joints and brazing agents. This fabrication technique is a production method that gives solid SiC reactors (thermal expansion coefficient = 4. 1x10⁻⁶K⁻¹).

Designed for flow rates ranging from 0.2 to 20 ml/minand pressures up to 25 bar, Protrix® allows the user to develop continuous flow processes at the lab-scale, later transitioning to Plantrix® MR555 (x340 scale factor) for material production. The Protrix® reactor is a unique flow reactor with the following advantages: diffusion bonded 3M® SiC modules with integrated heat exchangers that offer unrivaled thermal control and superior chemical resistance; safe employment of extreme reaction conditions on a g scale in a standard fumehood; efficient, flexible production in terms of number of reagent inputs, capacity or reaction time. The general specifications for the Protrix® flow reactors are summarised as follows; possible reaction types are, e.g. A + B → P1 + Q (or C) → P, wherein the terms "A", "B" and "C" represent educts, "P" and "P1" products, and "Q" quencher; throughput (ml/min) of from about 0.2 up to about 20; channel dimensions (mm) of1 x 1 (pre-heat and mixer zone), 1.4 x 1.4 (residence channel); reagent feeds of 1 to 3; module dimensions (width x height) (mm) of 110 x 260; frame dimensions (width x height x length) (mm) approximately 400 x 300 x 250; number of modules/frame is one (minimum) up to four (max.). More technical information on the ChemtrixProtrix® reactor can be found in the brochure "CHEMTRIX - Scalable Flow Chemistry - Technical Information Protrix®, published by Chemtrix BV in 2017, which technical information is incorporated herein by reference in its entirety.

The Dow Corning as Type G1SiC microreactor, which is scalable for industrial production, and as well suitable for process development and small production can be characterized in terms of dimensions as follows: typical reactor size (length x width x height) of 88 cm x 38 cm x 72 cm; typical fluidic module size of 188 mm x 162 mm. The features of the Dow Corning as Type GISiC microreactor can be summarized as follows: outstanding mixing and heat exchange: patented HEART design; small internal volume; high residence time; highly flexible and multipurpose; high chemical durability which makes it suitable for high pH compounds and especially hydrofluoric acid; hybrid glass/SiC solution for construction material; seamless scale-up with other advanced-flow reactors. Typical specifications of the Dow Corning as Type G1SiC microreactor are as follows: flow rate of from about 30 ml/min up to about 200 ml/min; operating temperature in the range of from about -60 °C up to about 200 °C, operating pressure up to about 18 barg ("barg" is a unit of gauge pressure, i.e. pressure in bars above ambient or atmospheric pressure); materials used are silicon carbide, PFA (perfluoroalkoxy alkanes), per fluoroelastomer; fluidic module of 10 ml internal volume; options: regulatory authority certifications, e.g. FDA or EMA, respectively. The reactor configuration of Dow Corning as Type GISiC microreactor is characterized as multipurpose and configuration can be customized. Injection points may be added anywhere on the said reactor.

Hastelloy® C is an alloy represented by the formula NiCr21Mo14W, alternatively also known as "alloy 22" or "Hastelloy® C-22. The said alloy is well known as a highly corrosion resistant nickel-chromium-molybdenum-tungsten alloy and has excellent resistance to oxidizing reducing and mixed acids. The said alloy is used in flue gas desulphurization plants, in the chemical industry, environmental protection systems, waste incineration plants, sewage plants. Apart from the before said example, in other embodiments of the invention, in general nickel-chromium-molybdenum-tungsten alloy from other manufactures, and as known to the skilled person, of course can be employed in the present invention. A typical chemical composition (all in weight-%) of such nickel-chromium-molybdenum-tungsten alloy is, each percentage based on the total alloy composition as 100 %: Ni (nickel) as the main component (balance) of at least about 51.0 %, e.g. in a range of from about 51.0 % to about 63.0 %; Cr (chromium) in a range of from about 20.0 to about 22.5 %, Mo (molybdenum) in a range of from about 12.5 to about 14.5 %, W (tungsten or wolfram, respectively) in a range of from about 2.5 to about 3.5 %; and Fe (iron) in an amount of up to about 6.0 %, e.g. in a range of from about 1.0 % to about 6.0 %, preferably in a range of from about 1.5 % to about 6.0 %, more preferably in a range of from about 2.0 % to about 6.0 %. Optionally, the percentage based on the total alloy composition as 100 %, Co (cobalt) can be present in the alloy in an amount of up to about 2.5 %, e.g. in a range of from about 0.1 % to about 2.5 %. Optionally, the percentage based on the total alloy composition as 100 %, V (vanadium) can be present in the alloy in an amount of up to about 0.35 %, e.g. in a range of from about 0.1 % to about 0,35 %. Also, the percentage based on the total alloy composition as 100 %, optionally low amounts (i.e. ≤ 0.1 %) of other element traces, e.g. independently of C (carbon), Si (silicon), Mn (manganese), P (phosphor), and/or S (sulfur). In such case of low amounts (i.e. ≤ 0.1 %) of other elements, the said elements e.g. of C (carbon), Si (silicon), Mn (manganese), P (phosphor), and/or S (sulfur), the percentage based on the total alloy composition as 100 %, each independently can be present in an amount of up to about 0.1 %, e.g. each independently in a range of from about 0.01 to about 0.1 %, preferably each independently in an amount of up to about 0.08 %, e.g. each independently in a range of from about 0.01 to about 0.08 %. For example, said elements e.g. of C (carbon), Si (silicon), Mn (manganese), P (phosphor), and/or S (sulfur), the percentage based on the total alloy composition as 100 %, each independently can be present in an amount of, each value as an about value: C ≤ 0.01 %, Si ≤ 0.08 %, Mn ≤ 0.05 %, P ≤ 0.015 %, S ≤ 0.02 %. Normally, no traceable amounts of any of the following elements are found in the alloy compositions indicated above: Nb (niobium), Ti (titanium), A1 (aluminum), Cu (copper), N (nitrogen), and Ce (cerium).

Hastelloy® C-276 alloy was the first wrought, nickel-chromium-molybdenum material to alleviate concerns over welding (by virtue of extremely low carbon and silicon contents). As such, it was widely accepted in the chemical process and associated industries, and now has a 50-year-old track record of proven performance in a vast number of corrosive chemicals. Like other nickel alloys, it is ductile, easy to form and weld, and possesses exceptional resistance to stress corrosion cracking in chloride-bearing solutions (a form of degradation to which the austenitic stainless steels are prone). With its high chromium and molybdenum contents, it is able to withstand both oxidizing and non-oxidizing acids, and exhibits outstanding resistance to pitting and crevice attack in the presence of chlorides and other halides. The nominal composition in weight-% is, based on the total composition as 100 %: Ni (nickel) 57 % (balance); Co (cobalt) 2.5 % (max.); Cr (chromium) 16 %; Mo (molybdenum) 16 %; Fe (iron) 5 %; W (tungsten or wolfram, respectively) 4 %; further components in lower amounts can be Mn (manganese) up to 1 % (max.); V (vanadium) up to 0.35 % (max.); Si (silicon) up to 0.08 % (max.); C (carbon) 0.01 (max.); Cu (copper) up to 0.5 % (max.).

In another embodiments of the invention, without being limited to, for example, the microreactor suitable for the said production, preferably for the said industrial production, is an SiC-microreactor that is comprising or is made only of SiC as the construction material (silicon carbide; e.g. SiC as offered by Dow Corning as Type G1SiC or by Chemtrix MR555 Plantrix), e.g. providing a production capacity of from about 5 up to about 400 kg per hour.

It is of course possible according to the invention to use one or more microreactors, preferably one or more SiC-microreactors, in the production, preferably in the industrial production, of the fluorinated products according to the invention. If more than one microreactor, preferably more than one SiC-microreactors, are used in the production, preferably in the industrial production, of the fluorinated products according to the invention, then these microreactors, preferably these SiC-microreactors, can be used in parallel and/or subsequent arrangements. For example, two, three, four, or more microreactors, preferably two, three, four, or more SiC-microreactors, can be used in parallel and/or subsequent arrangements.

For laboratory search, e.g. on applicable reaction and/or upscaling conditions, without being limited to, for example, as a microreactor the reactor type Plantrix of the company Chemtrix is suitable. Sometimes, if gaskets of a microreactor are made out of other material than HDPTFE, leakage might occur quite soon after short time of operation because of some swelling, so HDPTFE gaskets secure long operating time of microreactor and involved other equipment parts like settler and distillation columns.

For example, an industrial flow reactor ("IFR", e.g. Plantrix® MR555) comprises of SiC modules (e.g. 3M® SiC) housed within a (non-wetted) stainless steel frame, through which connection of feed lines and service media are made using standard Swagelok fittings. The process fluids are heated or cooled within the modules using integrated heat exchangers, when used in conjunction with a service medium (thermal fluid or steam), and reacted in zig-zag or double zig-zag, meso-channel structures that are designed to give plug flow and have a high heat exchange capacity. A basic IFR (e.g. Plantrix® MR555) system comprises of one SiC module (e.g. 3M® SiC), a mixer ("MRX") that affords access to A + B → P type reactions. Increasing the number of modules leads to increased reaction times and/or system productivity. The addition of a quench Q/C module extends reaction types to A + B → P1 + Q (or C) → P and a blanking plate gives two temperature zones. Herein the terms "A", "B" and "C" represent educts, "P" and "P1" products, and "Q" quencher.

Typical dimensions of an industrial flow reactor ("IFR", e.g. Plantrix® MR555) are, for example: channel dimensions in (mm) of 4 x 4 ("MRX", mixer) and 5 × 5 (MRH-I/MRH-II; "MRH" denotes residence module); module dimensions (width x height) of 200 mm x 555 mm; frame dimensions (width x height) of 322 mm x 811 mm. A typical throughput of an industrial flow reactor ("IFR", e.g. Plantrix® MR555) is, for example, in the range of from about 50 1/h to about 400 1/h. in addition, depending on fluid properties and process conditions used, the throughput of an industrial flow reactor ("IFR", e.g. Plantrix® MR555), for example, can also be > 400 1/h. The residence modules can be placed in series in order to deliver the required reaction volume or productivity. The number of modules that can be placed in series depends on the fluid properties and targeted flow rate.

Typical operating or process conditions of an industrial flow reactor ("IFR", e.g. Plantrix® MR555) are, for example: temperature range of from about -30 °C to about 200 °C; temperature difference (service - process) < 70 °C; reagent feeds of 1 to 3; maximum operating pressure (service fluid) of about 5 bar at a temperature of about 200 °C; maximum operating pressure (process fluid) of about 25 bar at a temperature of about ≤ 200 °C.

### Fluorosulfonylisocyanate may be prepared via FCN, as displayed in Scheme A:

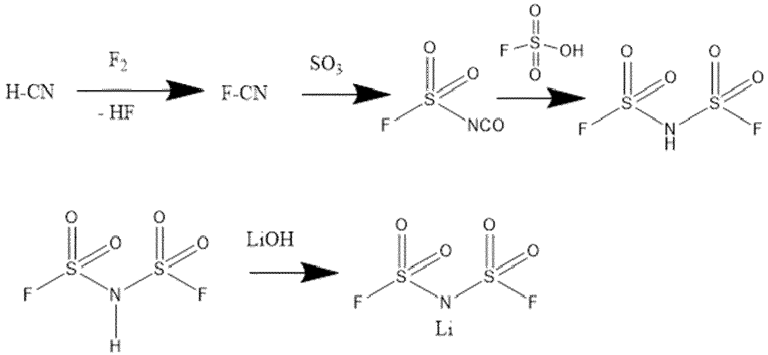

The fluorination product fluorocyanide (F-CN) can be used as raw material to prepare a conducting salt for lithium ion batteries.

However, regarding the scope of the present invention it is to be noted that, that for legal reason only but not for technical reason, there is a proviso that solely the use of fluorocyanide (F-CN) in the manufacture of a conducting salt for lithium ion batteries, solely any process for the manufacture of a conducting salt for lithium ion batteries derived from fluorocyanide (F-CN), and the conducting salt for lithium ion batteries itself derived from such use or manufacture including in at least one step fluorocyanide (F-CN), are encompassed by the scope of the present invention. The scope of the present invention, therefore, does not include the manufacture of fluorocyanide (F-CN) by direct fluorination with a fluorination gas comprising or consisting of a highly concentrated F₂-gas.

### Trifluorosulfonylisocyanate may be prepared over CF₃CN, as displayed in Scheme B, analogously and under analogous reaction conditions as for the preparation via FCN:

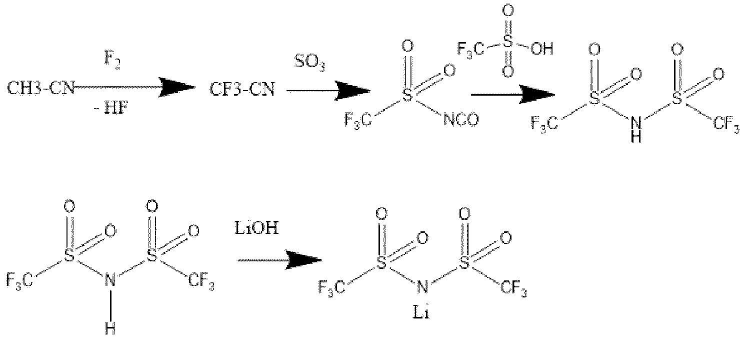

### Trifluoroacetonitrile (CF₃CN) can be prepared as shown in Scheme C:

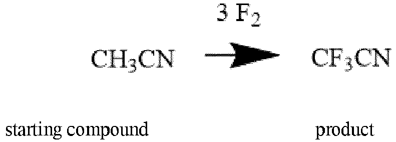

The fluorination product trifluoroacetonitrile (CF₃CN) can be used as raw material to prepare a conducting salt for lithium ion batteries of the present invention.

However, regarding the scope of the present invention it is to be noted that, that for legal reason only but not for technical reason, there is a proviso that solely the use of trifluoroacetonitrile (CF₃CN) in the manufacture of a conducting salt for lithium ion batteries, solely any process for the manufacture of a conducting salt for lithium ion batteries derived from trifluoroacetonitrile (CF₃CN), and the conducting salt for lithium ion batteries itself derived from such use or manufacture including in at least one step trifluoroacetonitrile (CF₃CN), are encompassed by the scope of the present invention. The scope of the present invention, therefore, does not include the manufacture of trifluoroacetonitrile (CF₃CN) by direct fluorination with a fluorination gas comprising or consisting of a highly concentrated F₂-gas.

### Trifluorosulfonylfluoride (CF₃SO₂F) can be prepared as shown in Scheme D:

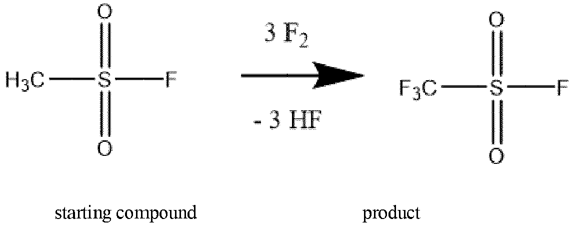

The fluorination product trifluorosulfonylfluoride (CF₃SO₂F) can be used as raw material to prepare a conducting salt for lithium ion batteries.

However, regarding the scope of the present invention it is to be noted that, that for legal reason only but not for technical reason, there is a proviso that solely the use of trifluorosulfonylfluoride (CF₃SO₂F) in the manufacture of a conducting salt for lithium ion batteries, solely any process for the manufacture of a conducting salt for lithium ion batteries derived from trifluorosulfonylfluoride (CF₃SO₂F), and the conducting salt for lithium ion batteries itself derived from such use or manufacture including in at least one step trifluorosulfonylfluoride (CF₃SO₂F), is encompassed by the scope of the present invention. The scope of the present invention, therefore, does not include the manufacture of trifluorosulfonylfluoride (CF₃SO₂F) by direct fluorination with a fluorination gas comprising or consisting of a highly concentrated F₂-gas.

### Microreactor Process of Direct Fluorination:

The Reactions in Schemes A to D also may pertain to a process for the manufacture of a fluorinated compound by direct fluorination, wherein the process is a continuous process, preferably wherein the continuous process is carried out in a microreactor.

Regarding the scope of the present invention it is to be noted that, that for legal reason only but not for technical reason, there is a proviso that any procedure or manufacture of a fluorinated, direct or indirect, precursor compound involving a direct fluorination of compounds with highly concentrated fluorine gas, unless expressively otherwise stated herein, and fluorinated compounds as such being directly obtained from such direct fluorination of compounds with highly concentrated fluorine gas are exempted from the scope of the present invention.

However, to be clear, in contrast to the above, the use a fluorinated, direct or indirect, precursor compound being directly or indirectly obtained by a direct fluorination of compounds with highly concentrated fluorine gas, i.e. the use in a process for the manufacture of fluorinated conductive salts for lithium ion batteries (Li-ion batteries) is expressively encompassed by the present invention.

Direct Fluorination: Introducing one or more fluorine atoms into a compound by chemically reacting a starting compound, e.g. according to the present invention an inorganic starting compound or organic starting compound, respectively, with elemental fluorine (F₂) such that one or more fluorine atoms are covalently bound into the fluorinated inorganic compound or organic compound, respectively.

A highly concentrated fluorine gas is to be understood for the purpose of this invention to be a fluorine gas (fluorination gas) with concentrations of substantially more than, in particular very much higher than 15 % by volume or in particular than 20 % by volume of elemental fluorine (F₂), especially of equal to much higher than 25 % by volume (i.e., at least 25 % by volume) of elemental fluorine (F₂), preferably of equal to much higher than 35 % by volume or in particular than 45 % by volume of elemental fluorine (F₂), that is used for chemical synthesis, especially for the manufacture or for preparation of a fluorinated inorganic compound or fluorinated organic compound, respectively, as final products and/or intermediates.

Herein, the direct fluorination is particularly making use of a fluorination gas, wherein the elemental fluorine (F₂) is present in a high concentration, and to a process for the manufacture of a fluorinated inorganic compound or a fluorinated organic compound, respectively, by direct fluorination employing a fluorination gas, wherein the elemental fluorine (F₂) is present in a high concentration.

For exampleuse is made of a fluorination gas, wherein the elemental fluorine (F₂) is present in a high concentration, for example, in a concentration of elemental fluorine (F₂) especially of equal to much higher than 15 % or 20 % by volume (i.e., at least 15 % or 20 % by volume), and preferably at least 25 % by volume, to a process for the manufacture of a fluorinated inorganic compound or a fluorinated organic compound, respectively, by direct fluorination employing a fluorination gas, wherein the elemental fluorine (F₂) is present in a high concentration.

The fluorination process of the invention may be performed batch-wise or in a continuous manner. If the process of the invention is performed batch-wise, a column (tower) reactor may be used. If the process of the invention is continuous a microreactor may be used. If desired, it is also possible to perform the process of the invention continuously in a column (tower) reactor (gas scrubber system). However, it is preferred to perform a continuous process of the invention in a microreactor.

Especially, in one aspect use may be made of a fluorination gas, wherein elemental fluorine (F₂) is present in a high concentration of substantially more than, in particular very much more than at least 10 % by volume of elemental fluorine (F₂), especially of equal to much higher than 15 % or 20 % by volume (i.e., at least 15 % or 20 % by volume), and preferably at least 25 % by volume, for the manufacture of a fluorinated inorganic compound or a fluorinated organic compound, respectively, in a liquid medium comprising or consisting of a starting compound having one or more hydrogen atoms that are capable of being substituted by means of a halogenation reaction, preferably wherein the fluorine (F₂) is present in the fluorine containing gas in a high concentration in a range of from substantially more than, in particular very much more than 15 % or 20 % by volume (i.e., at least 15 % or 20 % by volume), and preferably at least 20 % by volume, each up to 100 % by volume, preferably equal to or more than 25 % by volume and up to 100 % by volume (vol.-%).

It was found that, preferably in special equipment and with special reactor design such as, e.g., a microreactor or a packed bed tower (preferably made of Hastelloy), especially a packed bed tower containing fillers, e.g., metal fillers (e.g. Hastelloy) or plastic fillers, preferably wherein the tower (e.g., made out of Hastelloy) is filled either with E-TFE or metal fillings (Hastelloy), for example each of about 10 mm diameter as available from Raschig (http://www.raschig.de/Fllkrper). The type of fillings is quite flexible, Raschigs Pall-Rings made out of Hastelloycan be used, and advantageously E-TFE-fillings.

In the said special equipment and with special reactor design such as, e.g., a microreactor or a packed bed tower (preferably made of Hastelloy), a fluorine gas with concentrations of substantially more than, in particular very much higher than 15 % or 20 % by volume of elemental fluorine (F₂), especially of equal to much higher than 20 % by volume (i.e., at least 20 % by volume) of elemental fluorine (F₂), preferably of equal to much higher than 25 % by volume of elemental fluorine (F₂), can be used for chemical synthesis especially for the preparation of a fluorinated inorganic compound or a fluorinated organic compound, respectively, as final products and/or intermediates, for usage in agro-, pharma-, electronics-, catalyst, solvent and other functional chemical applications. This process allows fluorination chemistry with F₂ gas with concentrations preferably equal to substantially more than, in particular very much higher than 25 % by volume of elemental fluorine (F₂). In a applying the present fluorination process it is possible to perform chemistry with F₂ as it comes directly out of the F₂-electrolysis reactors (fluorine cells). A representative composition of fluorine gas produced by a fluorine cell is 97 % F₂, up to 3 % CF₄ (formed from damage of the electrodes), for example, traces of HF, NO₂, OF₂, OF₂, COF₂, each % by volume and based on the total volume of the fluorine containing gas as 100 % by volume.

In the fluorination gas the elemental fluorine (F₂) may be diluted by an inert gas. The inert gas then constitutes the substantial difference (e.g., there may be only minor quantities of by-products (e.g., CF₄) of no more than about 5 % by volume, preferably of no more than about 3 % by volume, and only traces impurities (e.g., such like HF, NO₂, OF₂, OF₂, COF₂), in the fluorination gas).

An inert gas is a gas that does not undergo chemical reactions under a set of given conditions. The noble gases often do not react with many substances and were historically referred to as the inert gases. Inert gases are used generally to avoid unwanted chemical reactions degrading a sample. These undesirable chemical reactions are often oxidation and hydrolysis reactions with the oxygen and moisture in air.

Typical inert gases are noble gases, and the very common inert gas nitrogen (N₂). The noble gases (historically also the inert gases; sometimes referred to as aerogens) make up a group of chemical elements with similar properties; under standard conditions, they are all odorless, colorless, monatomic gases with very low chemical reactivity. The six noble gases that occur naturally are helium (He), neon (Ne), argon (Ar), krypton (Kr), xenon (Xe), and the radioactive radon (Rn).

Purified argon and nitrogen gases are most commonly used as inert gases due to their high natural abundance (78.3% N2, 1% Ar in air) and low relative cost. The preferred is nitrogen (N₂) as the inert gas for diluting the elemental fluorine (F₂) in the fluorination gas to the desired but still high concentration, as defined herein.

In general, the fluorination gas containing the elemental fluorine (F2) is fed into the microreactor in accordance with the required stoichiometry (sometimes with a slight excess) for the targeted fluorinated product and fluorination degree, and adapted to the reaction rate.

The invention may employ more than a single microreactor, i.e., the fluorination process may employ two, three, four, five or more microreactors, for either extending the capacity or residence time, for example, to up to ten microreactors in parallel or four microreactors in series. If more than a single microreactor is employed, then the plurality of microreactors can be arranged either sequentially or in parallel, and if three or more microreactors are employed, these may be arranged sequentially, in parallel or both.

The fluorination processis also very advantageous, in one embodiment wherein the direct fluorination of the invention optionally is performed in a continuous flow reactor system, or preferably in a microreactor system.

In an preferred fluorination process, the fluorination reaction is carried out in at least one step as acontinuous processes, wherein the continuous process is performed in at least one continuous flow reactor with upper lateral dimensions of about ≤ 5 mm, or of about ≤ 4 mm,
preferably in at least one microreactor;
more preferably wherein the fluorination reaction is a continuous process in at least one microreactor under one or more of the following conditions:
- flow rate: of from about 10 ml/h up to about 400 1/h;
- temperature: of from about 30 °C up to about 150 °C;
- pressure: of from about 4 bar up to about 50 bar;
- residence time: of from about 1 second, preferably from about 1 minute, up to about 60 minutes.

In another preferred process the fluorination relates to such a process of preparing a compound according to the invention, wherein at least one of the said continuous flow reactors, preferably at least one of the microreactors, independently is a SiC-continuous flow reactor, preferably independently is an SiC-microreactor.

The following examples are intended to further illustrate the invention without limiting its scope.

### Examples

Following compounds or intermediates are prepared according to this invention.

LiFSI from SO₂Cl₂ over SO₂FCl and HF as F-source.

### Example 1:

### Synthesis of SO₂ClF without catalyst and without any presence of a fluorinating agent.

SO₂ClF is synthesized in batch by adding HF (anhydreous) into SO₂Cl₂, e.g., in a Roth autoclave with HDPTFE Inliner, 1 deep pipe and one outlet at gas phase (pressure kept at 10 bar) at room temperature (without any catalyst under evolvement of HC1 or continuosly in a microreactor system). So 110 g (0.82 mol) Sulfurylchlorid is filled into a 250 ml Autoclave (company Roth) having a HPTFE Inliner, after closing of the autoclave, 82.1 g (4.1 mol) anhydrous HF was added from a N₂ pressurized Cylinder from the liquid phase over the deep pipe into the autoclave. The autoclave is then slowly heated to 50°C with an oil bath (pressure kept at 10 bar) and formed HCl is let escape into a scrubber over a pressure valve.

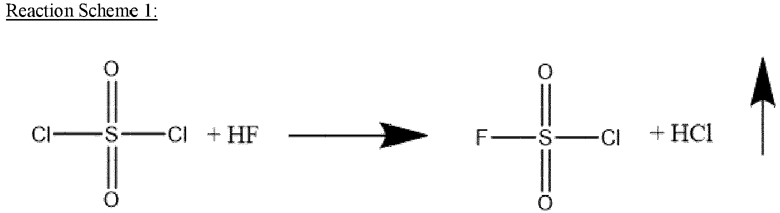

After 99.9 % of the HC1 has left the autoclave (calculated by analysis of Chloride in the scrubber), the remaining content (which is almost 100 % SO₂ClF + HF) is transferred into a stainless steel column and the SO₂ClF is distilled out of the HF at 5 bar, yield: 94 % (91 g). The higly volatile SO₂ClF (bp: 7°C) is put again into the autoclave, cooled to -30°C as preparation for the next step.

### Example 2:

### Conversion of SO₂ClF to FSO₂NH₂.

To the autoclave content which was 90 g (0.77 mol) as prepared under example 1,32.4 g (1.9 mol) NH₃ is now fed into the autoclave. The mixture is let to warm up and the reaction is finalized by keeping further 3 h at 50°C. The autoclave is cooled to room temperature, remaining pressure released into a scrubber and afterwards, the formed Ammoniumchloride is filtered off the formed Fluorosulfonylamide to get finally a yellow to brownisch liquid which is FSO₂NH₂ (97 % purity (GC)).

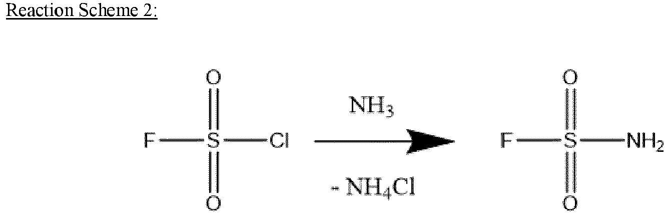

Remark: As SO₂ClF is much more reactive than SO₂Cl₂, the conversion to the isocyanate by using hazardous chemicals like in other literature references can be avoided and saved.

### Example 3:

### Conversion of FSO₂NH₂ to FSO₂NHSO₂F.

In the next step, added to the Fluorosulfonamide of example 2, is a 1.01 stochiometric amount of another ClSO₂F (prepared according to example 1) followed by a 1.01 stöchiometric amount of NH₃ which is kept together at 50°C for 2 h. After cooling down and expanding to atmospheric pressure, the formed NH₄Cl is filtered off again. A brown solid is isolated which is characterized as Bis-Fluorosulfonylimide. Other easier than NH₃ to handle bases like NEts, DBN, DBU, DMAP, Pyridine are also possible or could be added as accelerator but this alternatives are more difficult to be separated from the product.

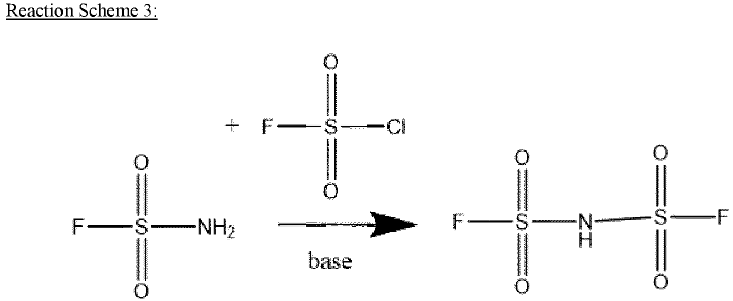

### Example 4:(inventive)

### Formation of LiFSI in alcohol.

It is knowledge of an expert that Bis-sulfonylimides are not stable vs. alcohols especially not in precence of a base as they usually can form Sulfonic acid esters. It is also known that water is difficult to be removed from salts especially like the ones out of sulfonylimides/bis-imides.

So the (raw) bis-sulfonylimide prepared in example 3 is dissolved in Isopropanol in a glass flask containing 2 fold stochiometric amount of LiOH. After 3 h at 75°C, the Isopropanol was removed with the stochometric amount of formed water in vacuum.

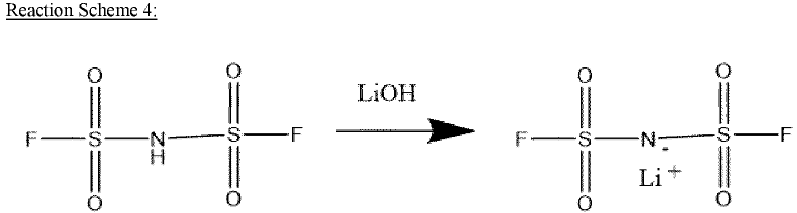

The achieved yield of LiFSI after another recrystallisation out of Isopropanol (example 3 + 4) was 159 g LiFSI with 99.99 % purity and a melting point of 128-130°C.

### Example 5:(inventive)

Example 4 was repeated using Li₂CO₂.

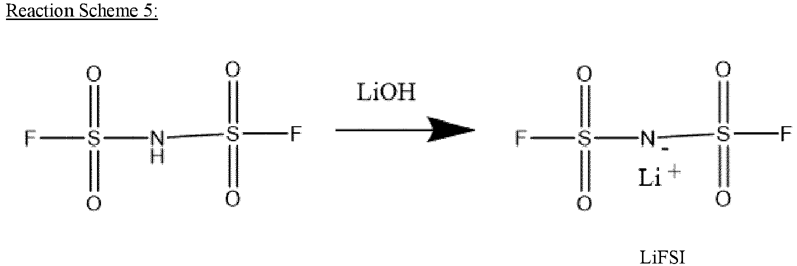

The reactivity of Li₂CO₃ vs. the Bis-fluorosulfonylimide is slower compared to LiOH. The reaction time was increased to 8 h.The Isopropanol was removed in vacuum and the residue re-cristallized in Isopropanol. The achieved yield was 89 %, the purity 99.8 %.

### Example 6:(inventive)

### LiFSI formation with LiF as Li-source.

LiF is commonly available and also a candidate as Li-source. Draw back of this route is that additionally another base like KOH (or others) is needed.

So KOH was dissolved in Isopropanol according to example 4 and reacted in 8 h with Bis-fluorosulfonylimide. The Potassium salt was isolated after removal of the solvent. The Isopropanol/water was removed in vacuum and the Potassium-bis-fluorosulfonylimide dissolved a 2^{nd} time in Isopropanol and treated with 1.5 fold excess of LiF. The yield in LiFSI was 82 %, purity after re-cristallisation 98.6 %.

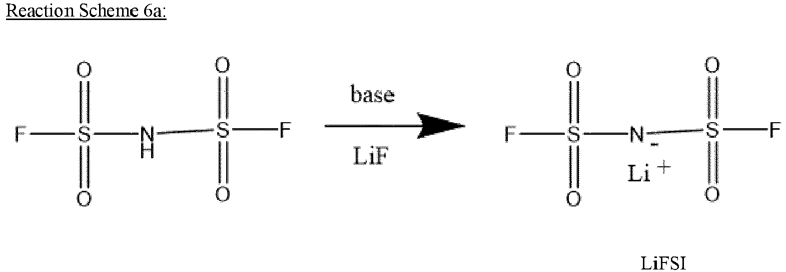

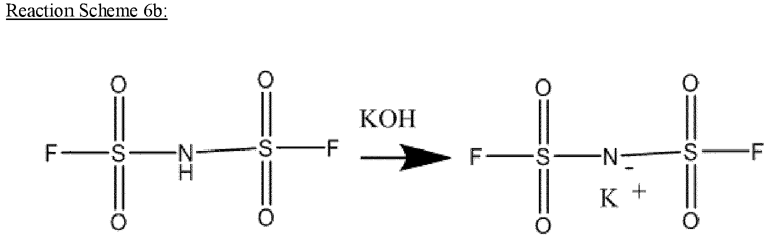

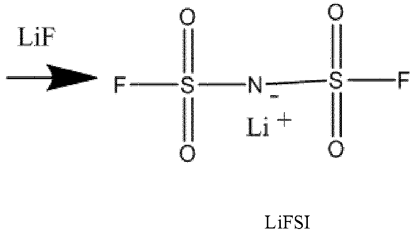

### Example 7:(inventive)

Synthesis of LiFSI with Li metal and conc. F₂ to achieve high purities. The synthesis was performed according to the flowing reaction Scheme 7a and 7b.

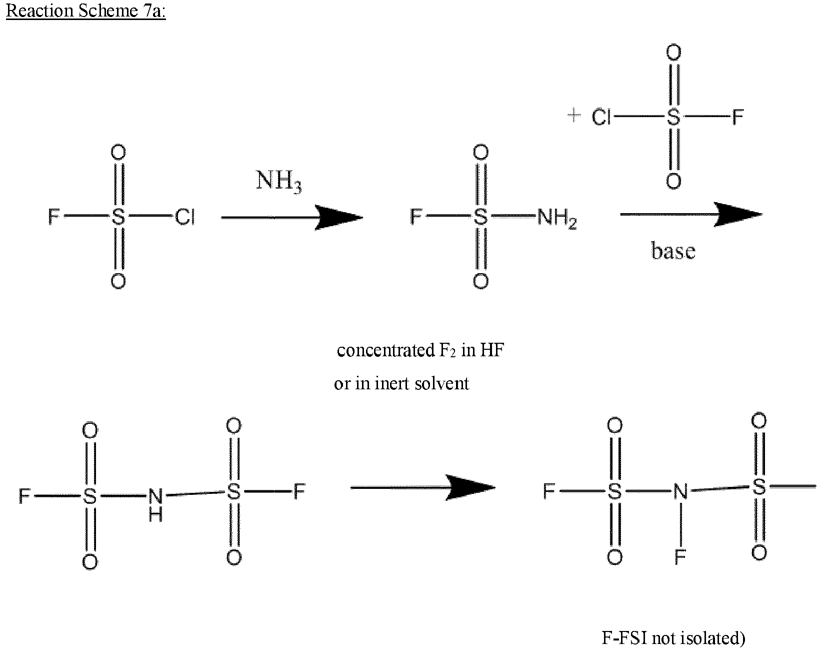

The F₂ fluorination cannot be applied in an earlier step but allow very high pure LiFSI after conversion with Li metal to form just LiF as a side product.

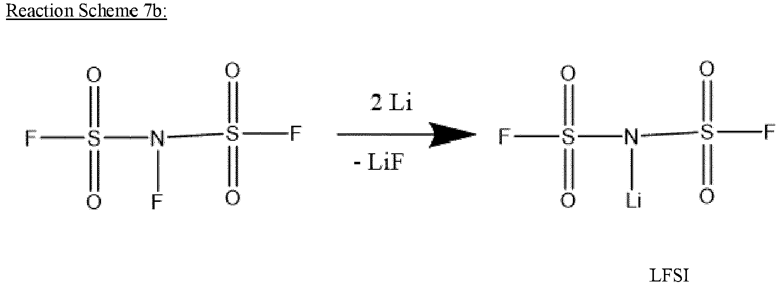

As bis-fluorosulfonyl imide and even more the corresponding salts are quite difficult to purify, treatment with F₂ gas followed by treatment with Li metal is another option.

120 g (0.66 mol) Bis-fluorosulfonylimide prepared according to example 3 is dissolved in Trifluorotoluene in a plastic apparatus and F₂-gas is fed out of a cylinder at room temperature over a Bronkhorst mass flow meter into the mixture until another mass flow meter at the exit of the apparatus shows same parameters as the input flow meter. The mixture was stirred for another hour at room temperature without F₂ feed to let some slight excess F₂ escape. Afterwards Li metal (4-16 mesh, Aldrich product No 228508) was very slowly! indroduced over a safety chamber/lock int the apparatur. A very strong exothermicity could be observed. Exactly 9.16 g (1.32 mol) Li was added while keeping a high speed of the stirrer and while the reaction temperature was kept at 20°C with a dry ice bath (CO₂) cooling from outside. The LiFSI was recrystallized from Isopropanol after filtering of the Li F by product and afterwards removing of the Triflurotoluene; achieved purity was > 99.99 %.

### Example 8: (inventive)

### Synthesis of LiFSI with LiH

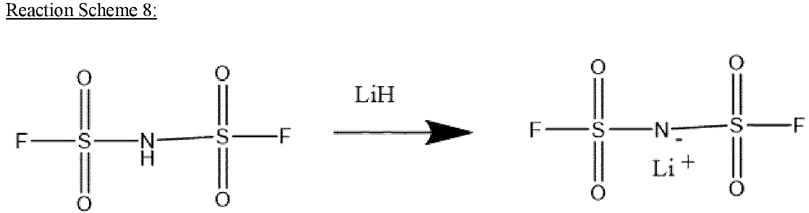

120 g (0.66 mol) Bis-fluorosulfonylimide prepared according to example 3 is dissolved in Trifluorotoluene in a glass flask. LiH (ABCR, product No AB117549) was very slowly introduced over a safety chamber/lock into the apparatus. A strong exothermicity together with H₂ evolution was observed. Exactly 10.69 g (0.77 ml) LiH was added with keeping a high speed of the stirrer and while the reaction temperature at 20°C with a dry ice bath (CO₂) from outside. The LiFSI was recrystallized from Isopropanol, the achieved purity was > 99.8 %.

### Example 9: (inventive)

### Example 9a: LiFSI overF-CN (analogous to C1CN).

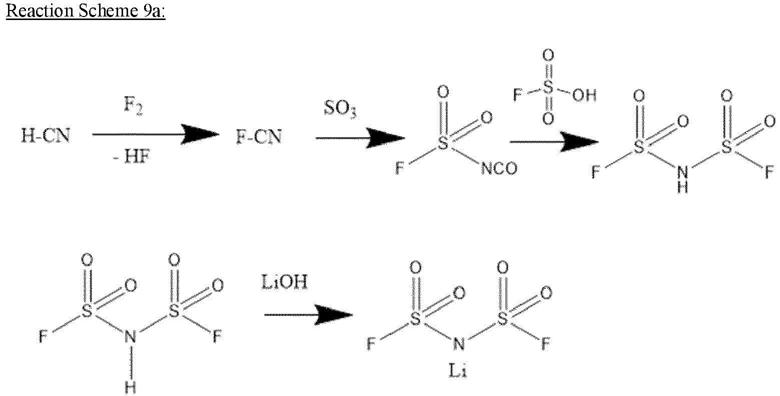

Advantage here is a very high purity of the Bis-fluorosulfonyl imide as the fluorine atom is in the molecule before formation of the amide. Fluorination of any Chlorosulfonylbis amide also gives the Bis-fluorosulfonylimide but all fluorinating agents and fluorination methods leave impurities which cannot or only with much efforts be removed. Very high purities are needed for usage as conducting salt in battery electrolytes.

(Cl-CN described with Cl₂ to C1CN WO 2014/065422 and Maugin, Ch.; Simon, L.-J.

In Annali di Chimica Applicata (1921)15, 18-41), F-CN only described out of Cl-CN by Seel, Fritz; Ballreich, Kurt in ChemischeBerichte (1959)92, 344-6 or 2,4,6-Trifluoro-s-triazine by Wu, Y.-Q. in Science of Synthesis (2005)18, 17-63).

LiFSI over chloro isocyanates (but not overfluororisocyanates) is described in WO 2009/123328) and WO 2017/080831.First information on the synthesis of LiFSI and corresponding triazole compounds are in US 7,919,629 and US 2011/0178306 and CA2527802.

The Sulfohalogenationof amines is described e.g. by Weiss, Guenther; Schulze, Gerhard in Justus Liebigs Annalen der Chemie (1969)729, 40-51, WO 2012/139775 and WO 2018/157240.

### Example 9b: LiTFSI over CF3-CN, analogous to LiFSI over F-CN.

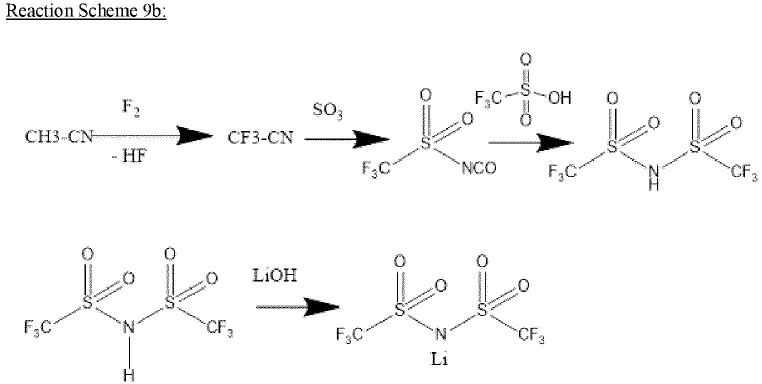

### Preparation is in analogy to the preparation of F-CN.

### Example 10: (comparative)

Synthesis of triethylammonium (fluorosulfonyl) trifluoromethanesulfonylimide salt as precursor for the Lithium (fluorosulfonyl) trifluoromethanesulfonylimide salt.

Comparative example from Central Glass WO 2016/080384 (paragraph 053).

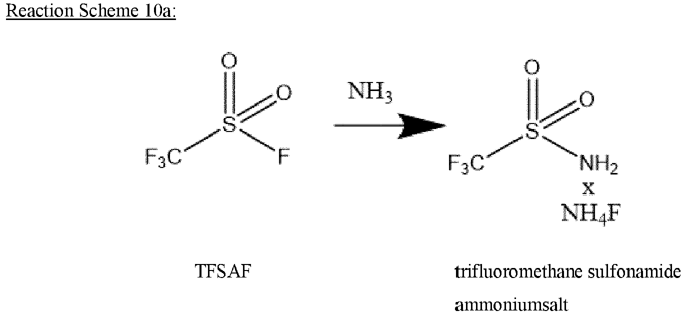

Reaction with NH₃: 300 g of acetonitrile was placed in a 500 ml stainless steel autoclave reactor, the reactor was cooled, and the inside of the reactor was evacuated when the liquid temperature became 5°C. or lower. After degassing, 24.7 g (1.45 mol) of anhydrous ammonia was added, and then 66.3 g (0.436 mol) of trifluoromethanesulfonyl fluoride (TFSAF) was slowly added while maintaining the internal temperature at 0 to 5 ° C. When introduction of trifluoromethanesulfonyl fluoride was completed, stirring was continued for 13 hours as it was. After 13 hours, the reaction solution was quantified by F-NMR, and as a result, 0.415 mol (yield: 95.1%) of trifluoromethanesulfonamide ammonium salt was obtained.

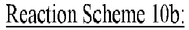

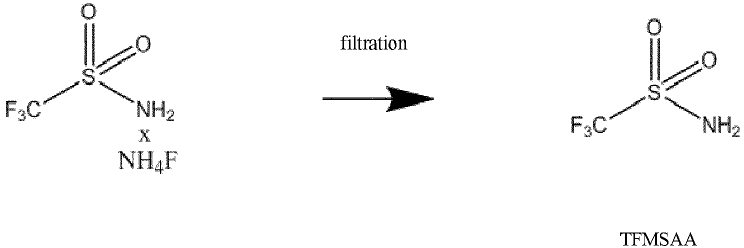

Filtration: After warming the reaction solution obtained in the first step to 40 ° C, filtration under reduced pressure was carried out using Kiriyama funnel. After filtration, the content of ammonium fluoride in the filtrate was confirmed by ion chromatography, and as a result, it was 0.0046 mol, and it was confirmed that it was able to be removed by 99%.

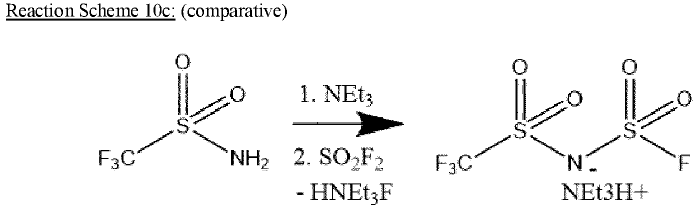

Reaction with base and SO₂F₂: The solution obtained in the step above is transferred to a reactor equipped with a Dimroth condenser tube, 126 g (1.25 mol) of triethylamine is added, and the solution is heated to 50 to 55 ° C. under reduced pressure of 0.064 to 0.068 MPa C for 12 hours to liberate ammonia. After the reaction, the content of ammonium ion was confirmed by ion chromatography, and it was confirmed that it decreased from 0.42 mol to 0.0007 mol (reduction rate of 99.8%). The solution obtained in the previous step was placed in a 1000 ml stainless steel autoclave reactor, the reactor was cooled, and the inside of the reactor was evacuated when the liquid temperature became 5 °C. or lower. After degassing, 63.15 g (0.623 mol) of sulfuryl fluoride was slowly added while keeping the internal temperature at 0 to 5 ° C. When introduction of sulfuryl fluoride was completed, stirring was continued for 17 hours as it was. After 17 hours, the reaction solution was quantified by F NMR. As a result, 0.404 mol of triethylammonium (fluorosulfonyl) trifluoromethanesulfonylimide salt as a target substance was obtained in a yield of 92.3%.

In this patent from Central Glass, it is also mentioned that the triethylammonium (fluorosulfonyl) trifluoromethanesulfonylimide salt can be further converted e.g. with Li₂CO₃ or LiOH and other Metal derivatives as listed hereunder to the corresponding Li-salt or other metal derivatives (Li given as example):

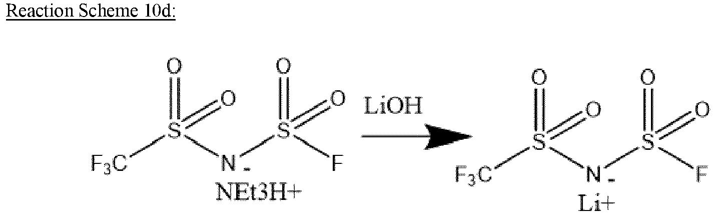

Examples for metals: As the alkali metal hydroxide to be used, alkali metal hydroxide such as lithium hydroxide (LiOH), sodium hydroxide (NaOH), potassium hydroxide (KOH), rubidium hydroxide (RbOH), cesium hydroxide (CsOH) As the salt, lithium carbonate (Li₂ CO₃), sodium carbonate (Na₂CO₃), potassium carbonate (K₂CO₃), rubidium carbonate (Rb₂CO₃), cesium carbonate (C_{S2}CO₃) As the metal hydroxide, magnesium hydroxide (Mg(OH)₂), calcium hydroxide (Ca(OH)₂), barium hydroxide (Ba(OH)₂), strontium hydroxide (Sr(OH)₂), Alkaline earth metal carbonate includes magnesium carbonate (MgCO₃), calcium carbonate (CaCO₃), barium carbonate (BaCO₃) , Strontium carbonate (SrCO₃), preferably lithium hydroxide (LiOH), sodium hydroxide (NaOH), potassium hydroxide (KOH), rubidium hydroxide (RbOH), cesium hydroxide (CsOH), hydroxide Magnesium (Mg (OH)₂), calcium hydroxide (Ca(OH)₂), barium hydroxide (BaOH₂). In the Central glass patent, an example for Na is given under paragraph 60.

Central glass recipe for M= Na: Next, 0.404 mol (134.2 g) of the triethylammonium (fluorosulfonyl) trifluoromethanesulfonylimide salt obtained in Example 1 and an aqueous solution containing 24.9 g of potassium hydroxide were mixed at room temperature and stirred for 1 hour did. After stirring, triethylamine and water contained in the reaction mixture were distilled off to obtain potassium (fluorosulfonyl) trifluoromethanesulfonylimide. Acetonitrile was further added thereto to remove undissolved components, and acetonitrile was distilled off to obtain 87.0 g of potassium (fluorosulfonyl) trifluoromethanesulfonylimide having a purity of 99% or more in a yield of 80%.

### Example 10e:(inventive)

The Example of Scheme 10c was repeated according to the invention, but the reaction was performed with F-SO₂-Cl and without NEt₃(base/catalyst).

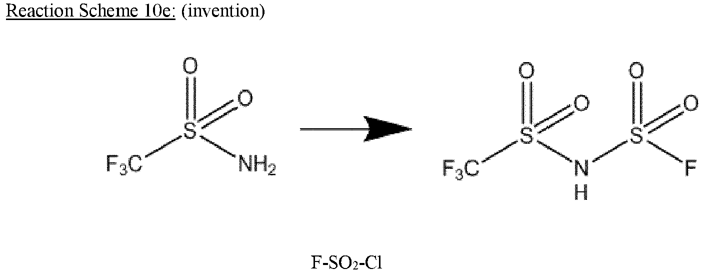

### Example 11: (comparative)

### LiTFSI synthesis

A modified easier procedure could be applied (as desired product is symmetric) and is disclosed for LiTFSi directly in DE 19533711 filed already on 12.9.1995 where NEt₃ is present in the mixture from the beginning of the reaction sequence. This has the advantage to save the step where NEt₃ is added after conversion with NH₃ and saves also the filtration step of NH₄F but it forms HNEt₃F instead. The reaction time for the 1 st step interestingly was only 3 h, it can be estimated that it was 13 h, may be.

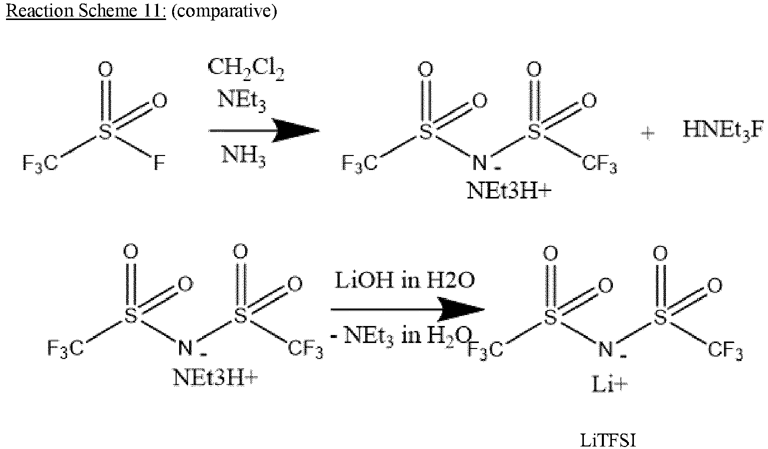

The achieved LiTFSI yield in DE 19533711 is mentioned with 94 % after dissolving in ether, removing not dissolvable impurities with Ether and removing the Ether.

The recipe of example 1 was repeated as given (13.5 g (0.133mol) NEt3, 20 g (0.132 mol) TFMSAF), the reaction time to the ammonium salt was 9 h instead of the given 3 h. The isolated yield after adding 3.2 g (0.133) LiOH in water for LiTFSI was 64 %.

### Example 12:(inventive)

Synthesis of LiTFSI using microreactor technology over Trifluoromethanesulfonic acid amide as separate step.

### a) Preparation of trifluoromethane sulfonic acid amide:

Trifluoromethane sulfonyl fluoride was used as raw material to prepare LiTFSI according to Reaction Scheme 12:

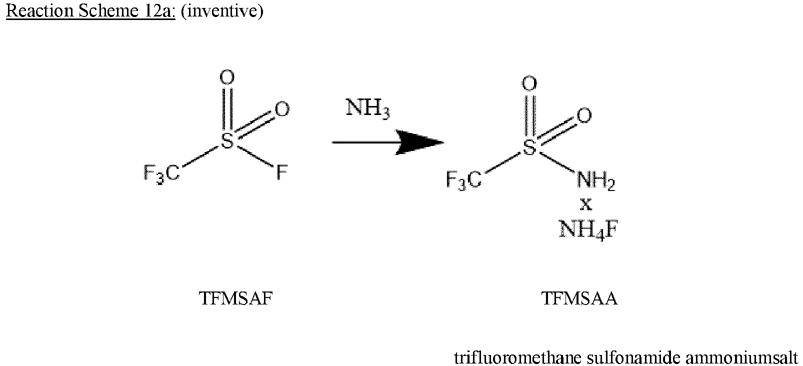

So into a 27 ml SiC-microreactor of Chemtrix (temperature 70°C), 100 g (0.66 mol) TFSAF and 34.06 g (2.0 mol) NH₃ were fed continuously per hour. The resulting mixture was collected in a steel cylinder after having passed a filter sieve to collect the NH₄F and to let the trifluoromethane sulfonamide (only with traces NH₄F) pass through.

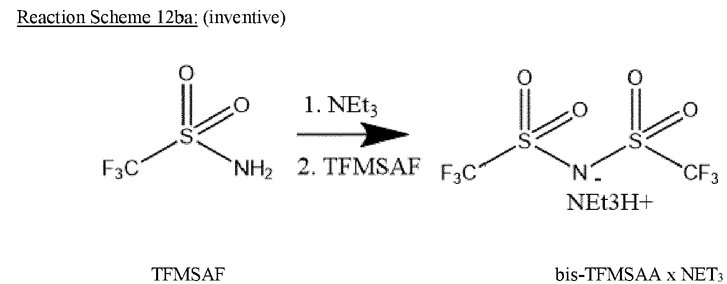

This filtered stream is fed together with 1.32 mol NEt₃ per hour in a first mixer and another 0.66 mol TFMSAF in another mixer into a 2nd microreactor (110°C) to give the triethylammoniumBis-(trifluoromethanesulfonyl)imide salt (Bis-TFMSAAxNEt₃) and HNEt₃F.

Reaction Scheme: LiTFSI over Trifluorosulfonic acid amide (TFMSAA) as isolated step. See Figure 1.

In a 3rd microreactor, the Bis-TFMSAAxNEt₃ is converted with 1 mol LiOH in water to LiTFSI which is further purified by simple re-crystallisation. It was found that NH₄F is somehow easier to be removed from the desired product than HNEt₃F so this reaction procedure has some purity advantage as overall HNEt₃F side product amount formed in 2^{nd}microreactor is than lower as half of the Fluoride leaving group coming from TFMSAF was already removed as NH₄F after microreactor 1 (NH₄F also has lower molecular weight if seen as as side product vs. NEt₃HF).

### Example 13:(inventive)

Simplified synthesis of LiTFSI using microreactor technology over direct synthesis of Bis-Trifluoromethanesulfonic acid imide triethylammonium salt (TFMSAA x NEts) and in situ synthesis of TFMSAA.

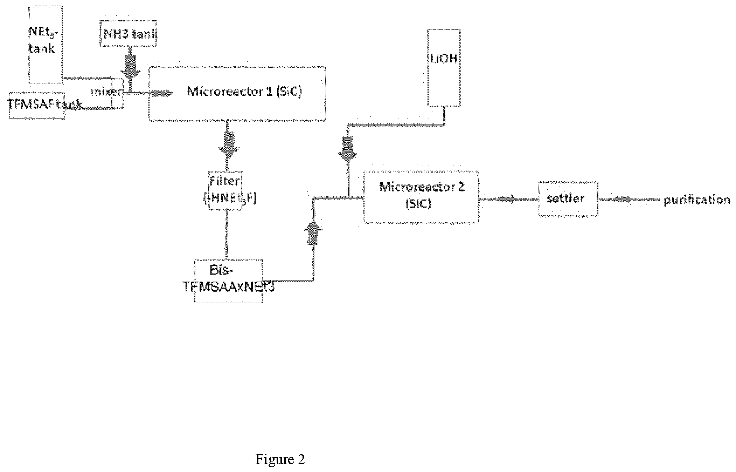

Reaction Scheme: LiTFSI over Triethylammomum-bis-(trifhioromethanesulfonyl)-imide salt (Bis-TFMSAAxNEt₃).See Figure 2.

So per hour into a 27 ml SiC-microreactor of Chemtrix (temperature 70°C), 200 g (1.32 mol) TFMSAF and 273.2 g (2.70 mol) NEt₃ in 100 ml CH₂Cl₂ were fed continuously over a split and recombine mixer together with 12.11 g (0.70 mol) NH₃. The resulting mixture was collected in a steel cylinder (not shown in the scheme above) after having passed a filter sieve to collect and remove the HNEt₃F and to let the triethylammoniumBis-(trifluoromethanesulfonyl)imide salt pass through. Afterwards LiOH in H2O is mixed into the product flow stream and fed into a 2^{nd}microreactor at 120°C to allow the formation of the LiTFSI together with NEt₃ in water which precipitate and which was isolated by a filtation step after the 2^{nd}microreactor and to let NEt₃ in water pass through. The isolated LiFSI was recrystallized up to a purity of 99.9 %.

### Example 14:(inventive)

Continuous preparation of LiFSI in microreactors.

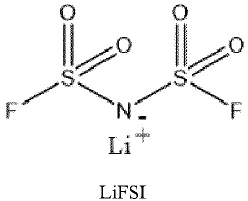

The chemistry as described in batch examples 1-5 were repeated and performed in microreactors according to following reaction scheme:
Reaction Scheme: Reaction Sequence with Four Microreactors. Steps 1-3 for Preparing LiFSI out of SO₂Cl₂ over SO₂ClF and over Fluorosulfonic Acid Amide and Bis-Fluorosulfonic acid imide.

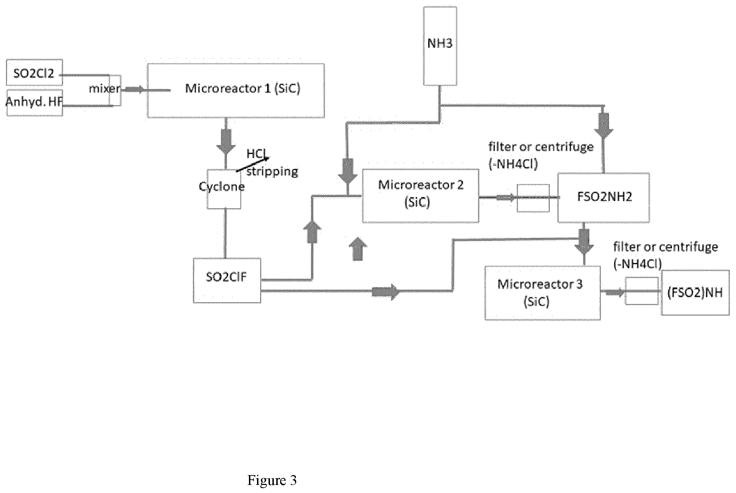

See Figure 3 (microreactors 1-3, reaction sequence until bis-fluorosulfonic acid imide).

Three-reactor design consisting out of threemicroreactors were operated to produce (FSO₂)NH (bis-fluorosulfonic acid imide).

A fourthmicroreactor can be used to convert continuously the bis-fluorosulfonic acid imide to the LiFSI. The Li-source is LiOH, LiH, Li₂CO₃. In the scheme the example is given for LiOH in water or Isopropanol. The construction material in the scheme is given with SiC, but Hastelloy and 1.4571 high grade stainless steel also is applicable. The SiC 27 ml microreactor from Chemtrix is used for microreactors no. 1 to no. 4. As the residence time is different (2 min in microreactorno. 1 at t = 70°C, 30 sec in microreactor 2 kept at 30°C and 3 also at 30°C (micoreactorno.2 and no. 3 are cooled), 20 min in microreactorno. 4 kept at 50°C) sufficient residence time is allowed by just multiplying microreactor modules with 27 ml.

Reaction Scheme: Reaction Sequence with Four Microreactors. Step 4 for Conversion of Bis-Fluorosulfonic Acid Imide into the Target Product LiFSI (out of SO₂Cl₂ over SO₂ClF and over Fluorosulfonic Acid Amide and Bis-Fluorosulfonic Acid Imide of Steps 1-3).See Figure 3 (microreactors 1-3, reaction sequence until Bis-Fluorosulfonic acid imide).The isolated yield LiTFSI based on SO₂Cl₂ over all steps was 94 %, the purity 99.9 % after the centrifuge and 99.99 % after additional recrystallization.

## Claims

1. A process for the manufacture of a fluorinated bis-sulfonyl imide of formula (I), preferably as precursor for a conductive lithium salt of the corresponding fluorinated sulfonyl imide of formula (II) for use in a lithium battery application, 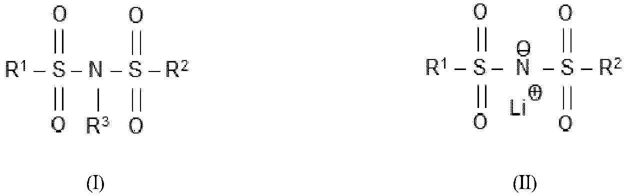 wherein in formula (I) and in formula (II),
R1 and R2 independently denote fluorine (F) or a trifluormethyl group, and
R3 denotes hydrogen (H), fluorine (F), or R3 denotes an electron pair forming together with the nitrogen (N) negatively charged nitrogen (N⁻) an to which in turn a positively charged tri-alkyl ammonium group is ionically bound, preferably a tri-ethyl ammonium group;
wherein the process comprises at least one or a combination of the following:
(i) providing as a first direct or indirect precursor compound of a fluorinated bis-sulfonyl imide of formula (I), a fluorinated sulfonyl compound having the formula (III),
F-SO₂-Cl (III),
(fluorosulfuric acid chloride), or a fluorinated sulfonyl compound selected from the group consisting of a compound having one of formulae (IV), (V) or (VI), with the proviso that the said one compound of formulae (IV), (V) or (VI) is directly obtained from the fluorinated sulfonyl compound having the formula (III),
F-SO₂-OH (IV),
CF₃-SO₂-OH (V),
CF₃-SO₂-Cl (VI);
and
(ii) providing as a second precursor compound of a fluorinated bis-sulfonyl imide of formula (I), a compound selected from the group consisting of a sulfonyl amino compound (sulfuric acid amide compound) a) to c), or a sulfonyl isocyanate compound d) to e):
a) F-SO₂-NH₂ (VII) derived from F-SO₂-NH₂ x NH4F, obtained by reacting F-SO₂-Cl with ammonia (NH₃), or
b) F₃C-SO₂-NH₂ (VIII) derived from F₃C-SO₂-NH₂ x NH₄F, obtained by reacting F₃C-SO₂-Cl with ammonia (NH₃), or
c) F₃C-SO₂-NH₂ (VIII) derived from F₃C-SO₂-NH₂ x NH₄F, obtained by reacting F₃C-SO₂-F (TFMSAF, trifluoromethylsulfuric acid fluoride) with ammonia (NH₃),
d) F-SO₂-N=C=O (IX), obtained by reacting F-CN with SO₃, or
e) F₃C-SO₂-N=C=O (X), obtained by reacting CF₃-CN with SO₃;
(iii) reacting a first precursor compound of (i) with a second precursor compound of (ii), each of the a fluorinated bis-sulfonyl imide of formula (I), with the proviso that the second precursor compound of (ii),
(1) in case of a), b) and c) the compound (VII) of a), the compound (VII) of b), or the compound (VII) of c) is reacted either with the fluorinated sulfonyl compound having the formula (III), F-SO₂-Cl, or with the fluorinated sulfonyl compound having the formula (the fluorinated sulfonyl compound having the formula (VI), CF₃-SO₂-Cl,
to obtain a fluorinated bis-sulfonyl imide of formula (I), wherein R3 denotes hydrogen (H), and R1 and R2 independently denote fluorine (F) or a trifluormethyl group;
or
(2) in case of d) and e), the compound (IX) of d) or the compound (X) of e) is reacted either with the fluorinated sulfonyl compound having the formula (IV), F-SO₂-OH, or with the fluorinated sulfonyl compound having the formula (the fluorinated sulfonyl compound having the formula (V), CF₃-SO₂-OH,
to obtain a fluorinated bis-sulfonyl imide of formula (I), wherein R3 denotes hydrogen (H), and R1 and R2 independently denote fluorine (F) or a trifluormethyl group;
or
(3) in case of a), b) and c) the compound (VII) of a), the compound (VII) of b), or the compound (VII) of c) is reacted with NEt₃ and TFMSAF (trifluoromethylsulfuric acid fluoride)
to obtain a fluorinated bis-sulfonyl imide of formula (I), wherein R3 denotes an electron pair forming together with the nitrogen (N) negatively charged nitrogen (N⁻) an to which in turn a positively charged tri-alkyl ammonium group is ionically bound, preferably a tri-ethyl ammonium group, preferably wherein R3 is NEt₃ and the fluorinated bis-sulfonyl imide of formula (I) is bis-TFMSAA x NEt₃;
and optionally
(4) further reacting the fluorinated bis-sulfonyl imide of formula (I) obtained under (1) or (2), wherein R3 is hydrogen (H), with concentrated elemental fluorine (F₂) in hydrogen fluoride (HF) or concentrated elemental fluorine (F₂) in an inert solvent,
to obtain a fluorinated bis-sulfonyl imide of formula (I), wherein R3 denotes fluorine (F);
and optionally
(5) converting the fluorinated bis-sulfonyl imide of formula (I), obtained under any of (1) to (4) into a conductive lithium salt of the corresponding fluorinated sulfonyl imide of formula (II), preferably the sulfonyl imide of formula (II) for use in a lithium battery application, wherein R1 and R2 independently denote fluorine (F) or a trifluormethyl group.

2. The process according to claim 1, for the manufacture of a fluorinated bis-sulfonyl imide of formula (I), wherein the process is carried out without a catalyst.

3. The process according to claim 1 or claim 2, for the manufacture of a fluorinated bis-sulfonyl imide of formula (I), wherein the process is carried out at least in one step in a microreactor.

4. The process according to any of the preceding claims, for the manufacture of a fluorinated bis-sulfonyl imide of formula (I), wherein the process is carried out with a first direct or indirect precursor compound of the a fluorinated bis-sulfonyl imide of formula (I), which is a fluorinated sulfonyl compound having the formula (III),
F-SO₂-Cl (III),
(fluorosulfuric acid chloride).

5. The process according to any of the preceding claims, for the manufacture of a fluorinated bis-sulfonyl imide of formula (I), wherein the process is carried out in step (ii) of claim 1 by providing as a second precursor compound of the a fluorinated bis-sulfonyl imide of formula (I), a compound selected from the group consisting of a sulfonyl amino compound (sulfuric acid amide compound) a) to c).

6. The process according to any of claim 1 to claim 4, for the manufacture of a fluorinated bis-sulfonyl imide of formula (I), wherein the process is carried out in step (ii) of claim 1 by providing as a second precursor compound of the a fluorinated bis-sulfonyl imide of formula (I), a compound selected from the group consisting of a sulfonyl isocyanate compound d) to e).

7. The process according to any of the preceding claims, for the manufacture of a fluorinated bis-sulfonyl imide of formula (I), wherein the fluorinated bis-sulfonyl imide of formula (I), is selected from the group consisting of:
| | | |
|---|---|---|
| LiFSI | lithium bis-(fluoromethanesulfonlyl) imide | 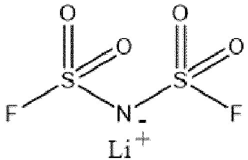 |
| | (Li bis(fluoromethanesulfonlylimide) salt) | |
| LiTFSI | lithium bis-(trifluoromethanesulfonlyl) imide | 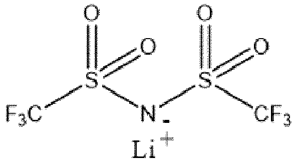 |
| | (Li bis-(trifluoromethanesulfonlyl) imide) salt) | |
| | | |
| LiTFSFI | lithium trifluoromethanesulfonylfluorosulfonyl imide | 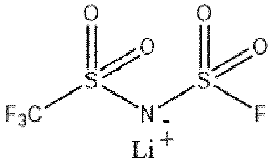 |
| | (Li trifluoromethanesulfonylfluorosulfonyl imide salt) | |

8. The process according to any of claims 1 to claim 7, for the manufacture of a fluorinated bis-sulfonyl imide of formula (I), wherein the fluorinated bis-sulfonyl imide of formula (I), is lithium bis-(fluoromethanesulfonlyl) imide (LiFSI, Li bis-(fluoro-methanesulfonlyl imide) salt).

9. The process for the manufacture of a fluorinated compound according to any of claims 1 to 8, wherein the reaction is carried out in at least one step as acontinuous processes, wherein the continuous process is performed in at least one continuous flow reactor with upper lateral dimensions of about ≤ 5 mm, or of about ≤ 4 mm,
preferably in at least one microreactor;
more preferably wherein of the said steps at least (b2) the step of a fluorination reaction is a continuous process in at least one microreactor under one or more of the following conditions:
- flow rate: of from about 10 ml/h up to about 400 1/h;
- temperature: of from about 30 °C up to about 150 °C;
- pressure: of from about 4 bar up to about 50 bar;
- residence time: of from about 1 second, preferably from about 1 minute, up to about 60 minutes.

10. A process of preparing a compound according to claim 9, wherein at least one of the said continuous flow reactors, preferably at least one of the microreactors, independently is a SiC-continuous flow reactor, preferably independently is an SiC-microreactor.

11. A use of a precursor compound selected from the group consisting of:
(i) as a first direct or indirect precursor compound (1-i) or (1-ii) of a fluorinated bis-sulfonyl imide of formula (I);
(i-1) a fluorinated sulfonyl compound having the formula (III),
F-SO₂-Cl (III),
(fluorosulfuric acid chloride); or
(i-2) a fluorinated sulfonyl compound selected from the group consisting of a compound having one of formulae (IV), (V) or (VI), with the proviso that the said one compound of formulae (IV), (V) or (VI) is directly obtained from the fluorinated sulfonyl compound having the formula (III),
F-SO₂-OH (IV),
CF₃-SO₂-OH (V),
CF₃-SO₂-Cl (VI);
or
(ii) as a second precursor compound of a fluorinated bis-sulfonyl imide of formula (I), a compound selected from the group consisting of a sulfonyl amino compound (sulfuric acid amide compound) a) to c), or a sulfonyl isocyanate compound d) to e):
a) F-SO₂-NH₂ (VII) derived from F-SO₂-NH₂ x NH4F, obtained by reacting F-SO₂-Cl with ammonia (NH₃), or
b) F₃C-SO₂-NH₂ (VIII) derived from F₃C-SO₂-NH₂ x NH₄F, obtained by reacting F₃C-SO₂-Cl with ammonia (NH₃), or
c) F₃C-SO₂-NH₂ (VIII) derived from F₃C-SO₂-NH₂ x NH₄F, obtained by reacting F₃C-SO₂-F (TFMSAF, trifluoromethylsulfuric acid fluoride) with ammonia (NH₃),
d) F-SO₂-N=C=O (IX), obtained by reacting F-CN with SO₃, or
e) F₃C-SO₂-N=C=O (X), obtained by reacting CF₃-CN with SO₃;
in a process for the manufacture of a fluorinated bis-sulfonyl imide of formula (I), preferably as precursor for a conductive lithium salt of the corresponding fluorinated sulfonyl imide of formula (II) for use in a lithium battery application, 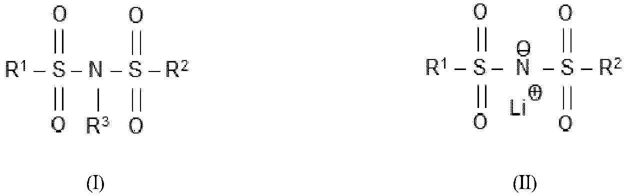 wherein in formula (I) and in formula (II),
R1 and R2 independently denote fluorine (F) or a trifluormethyl group, and
R3 denotes hydrogen (H), fluorine (F), or R3 denotes an electron pair forming together with the nitrogen (N) negatively charged nitrogen (N⁻) an to which in turn a positively charged tri-alkyl ammonium group is ionically bound, preferably a tri-ethyl ammonium group.

12. The use of a precursor compound according to claim 11, in a process for the manufacture of a fluorinated bis-sulfonyl imide of formula (I), preferably as precursor for a conductive lithium salt of the corresponding fluorinated sulfonyl imide of formula (II) for use in a lithium battery application,
wherein the use of at least one precursor compound in the process for the manufacture of a fluorinated compound of formula (I) or formula (II), as each defined in claim 11, takes place in a reaction which is carried out in at least one step as a continuous processes, wherein the continuous process is performed in at least one continuous flow reactor with upper lateral dimensions of about ≤ 5 mm, or of about ≤ 4 mm,
preferably in at least one microreactor;
more preferably wherein of the said steps at least (b2) the step of a fluorination reaction is a continuous process in at least one microreactor under one or more of the following conditions:
flow rate: of from about 10 ml/h up to about 400 1/h;
- temperature: of from about 30 °C up to about 150 °C;
- pressure: of from about 4 bar up to about 50 bar;
- residence time: of from about 1 second, preferably from about 1 minute, up to about 60 minutes.

13. The use of a precursor compound according to claim 12, wherein the use of at least one precursor compound in the process for the manufacture of a fluorinated compound of formula (I) or formula (II), as each defined in claim 11, takes place in a continuous process, wherein at least one of the said continuous flow reactors, preferably at least one of the microreactors, independently is a SiC-continuous flow reactor, preferably independently is an SiC-microreactor.
